# EUROPEAN PATENT SPECIFICATION

(11) **EP 4 243 686 B1**
(45) Date of publication and mention of the grant of the patent: **03.09.2025**
(21) Application number: 20815748.7
(22) Date of filing: 24.11.2020
(51) Int. Cl.: A61B 5/11, A61B 5/107, A61B 5/00

(54) **A METHOD OF GENERATING A REAL TIME PROTRACTOR DISPLAY**
VERFAHREN ZUR GENERIERUNG EINES ECHTZEIT PROTRAKTOR-DISPLAYS
PROCEDE DE GÉNÉRATION D'UN AFFICHAGE DE RAPPORTEUR EN TEMPS RÉEL

(30) Priority: 11.11.2020 GB 202017767
(43) Date of publication of application: 20.09.2023
(60) Divisional application: 24193163.3
(73) Proprietor: 3D4Medical Limited, D18 X6N2 Dublin (IE)
(72) Inventor: WALSH, Irene, Blackrock, Co Dublin 0 (IE); OPANASIUK, Mykhailo, Blackrock, Co Dublin 0 (IE); NOVIKOV, Sergey, Blackrock, Co Dublin 0 (IE)
(74) Representative: Franks & Co Limited
(86) International application number: PCT/EP2020/083237
(87) International publication number: WO 2022/100872

(56) References cited:
- WO-A1-2019/183733
- US-A1- 2012 249 416
- US-A1- 2017 368 413

## Description

### Field of the Invention

The present invention relates to a method for making goniometer measurements between anatomical parts.

### Background of the Invention

In assessment of medical patient's body flexibility and range of movement, it is known to use a goniometer to measure articulation of limbs, joints and flexation of the body.

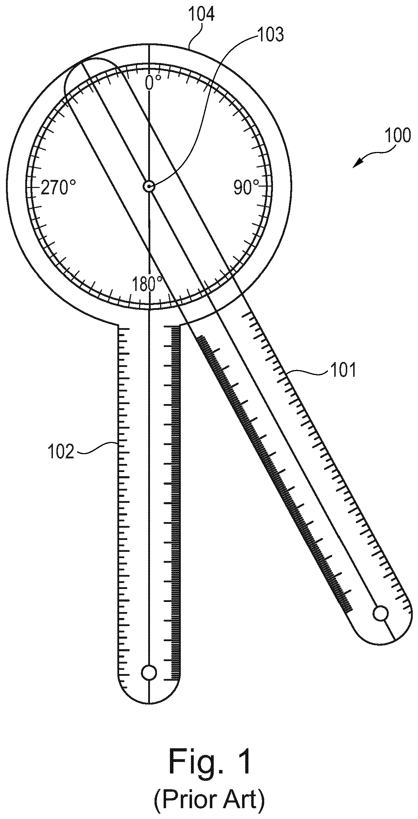

Referring to Figure 1 herein, a known physical goniometer 100 comprises a pair of elongate arms 101, 102 attached by a rotatable joint 103, each arm having a distance ruler scale to measure distance from a pivot point about which the arms are rotatable with respect to each other, and at one end of one of the arms there is provided a circular protractor 104 having a graduated circle marked with angles from a fixed datum point corresponding with a main central length axis of the first arm.

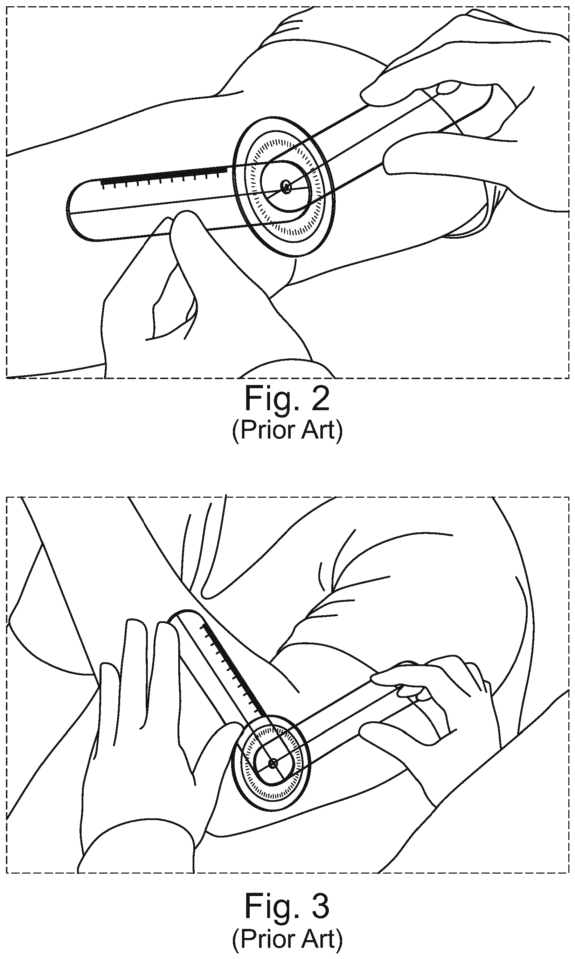

Referring to Figures 2 and 3 herein, in use, to measure the angle of flexation or movement of two body parts, for example a human elbow joint, a medical practitioner or clinician aligns the centre of the pivot point with a nominal centre of rotation of the elbow joint by estimation, and visually aligns a first arm of the goniometer with a main length direction of the upper arm, and visually aligns the second arm of the goniometer with a lower portion of the arm. The patient is asked to extend or bend their arm to the maximum extent possible and the medical practitioner re-aligns the first and second arms of the goniometer instrument with the main length directions of the upper and lower portions and visually takes an angle measurement by reading the angle between the two goniometer arms using the graduated protractor. To measure the maximum extent of articulation of the elbow joint, a first angle reading is taken at maximum extension of the patient's arm, and a second angle reading is taken at maximum bend of the patient's arm.

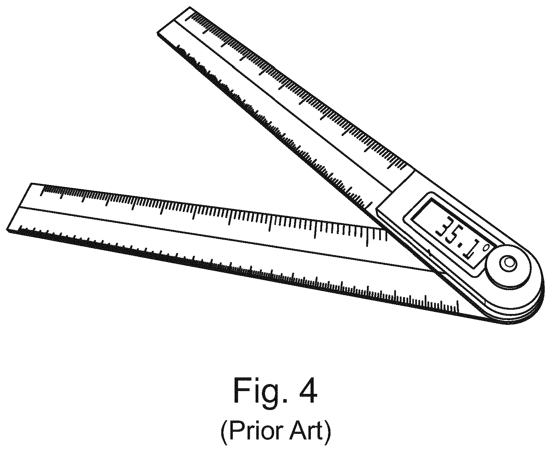

Referring to Figure 4 herein, there is illustrated schematically a known digital medical goniometer, which is a development of the traditional mechanical goniometer as shown in Figure 1. The digital goniometer has the advantage that instead of a physical circular plate protractor at the pivot point of the goniometer arms to measure the angle between the two arms, there is provided a digital electronic meter which measures the angle between the two goniometer arms and displays the angle on a liquid-crystal display.

Use of the known digital goniometer is similar to that described for the mechanical goniometer, but instead of reading a mechanical graduated scale of angle, the medical practitioner reads a liquid-crystal display of a digitally presented angle, which gives an order of magnitude greater accuracy in reading the angle between the arms compared to the mechanical goniometer.

There are also various known applications for use on handheld computing devices, including smartphones which provide a goniometer function and which measure range of motion of a body. These include the known, Rate Fast goniometer application and the known Goniometer Pro (G-pro) application.

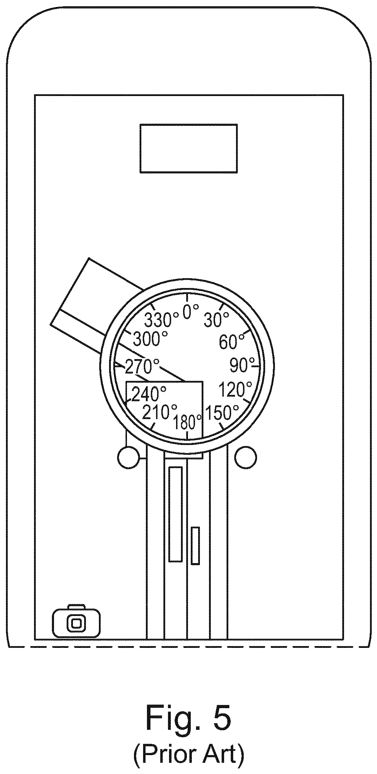

Referring to Figure 5 herein, there is illustrated schematically a screen view of the known RateFast goniometer application in one mode of operation in which the camera of a smartphone or other hand held computing device is used to take a video or still digital image of a limb to be measured. The goniometer application superimposes a picture of a mechanical goniometer onto an on-screen image viewed through the digital camera of the smart phone or other handheld computing device. The on-screen image of the goniometer is fixed relationship to the screen, and therefore to the smartphone. Initially a user may reference the attitude of the smartphone to the subject being measured by visually aligning a centre point of the goniometer image on screen with the centre of an image of a joint, by moving the smartphone around until the goniometer centre coincides with the centre of the joint, and where the main lower arm of the goniometer image is held substantially vertical. The user captures this initial orientation image, and the application also captures the initial orientation data of the smartphone to use as a reference orientation from the internal attitude sensors of the smartphone.

The user then aligns the main arm of the screen-presented goniometer with a main length of a first limb portion on one side of the joint, for example an upper arm portion. A first image is captured with the smartphone held in a first orientation, in which the smartphone is held in an orientation corresponding to a main length axis of the first body part on one side of the joint. The application uses data from an inclinometer or accelerometer in the smart phone to determine the orientation of the smart phone in the first orientation. The user then tilts the whole smart phone to align the main length of the on-screen goniometer image with a main length of a second body portion on the other side of a joint and captures a second image in a second orientation of the smartphone. The application measures the attitude of the smartphone from the inclinometer and/or accelerometer built-in within the smart phone to obtain a second angle/orientation relative to vertical.

The application then calculates the angular difference between the first and second orientations which corresponds with the angle of the joint being measured.

In another mode of operation, instead of aligning a screen image of a goniometer with a subject's limb portions or body portions either side of a joint, the user simply orientates the whole smart phone so that the length of the device is parallel to the main length of the body portion. For example to measure the angle of flexion of an elbow joint, the user would align the length of the screen of the smart phone with the main length of the upper arm portion and capture a first reading which causes the application to record the orientation data (angle to the vertical) from the accelerometer data of the accelerometer inbuilt in the smart phone, and then repeats the operation by aligning the screen of the smart phone with the main length of the lower arm portion on the other side of the joint to capture another reading, with the application recording the orientation of the smart phone from a reading of the orientation sensor or accelerometer within the smart phone.

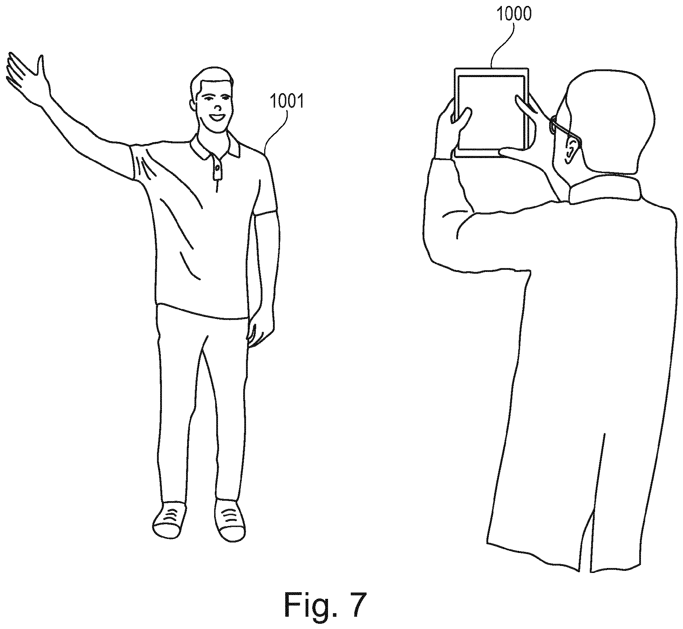

Referring to Figure 7 herein there is illustrated schematically a screen view of the known Goniometer-pro (G-pro) goniometer device, also for use with a smart phone or other hand held computing device that shows a user how to take a limb angle measurement by attaching the device to their arm. In the G-pro system angle measurements are derived from the internal accelerometer of the handheld device. This requires initial calibration of the device in a known initial attitude or orientation, and subsequent determination of an angle of movement based upon the measurement of acceleration and deceleration from the known initial orientation.

The known prior art goniometers have the following problems. In the case of the mechanical goniometer errors occur through misalignments of the length of the mechanical arms with the length of the limb portion, and there is a limitation to the accuracy of the visual reading of angle from the mechanical protractor. The user needs to be in close proximity to the subject and cannot view the subject's posture from a distance. Also, more than one type of goniometer may be needed. There may be required a series of goniometers operating on different scales to cover the different scales of movement of the body, for example for the swinging of hips and for flexation of fingers. Both larger and smaller scale goniometers may be needed for different parts of the anatomy.

In the case of the digital goniometer of Figure 4, there is improved measurement of angle between the two arms of the goniometer, but accuracy of angle measurement is still limited by the alignment of the arms of the goniometer with the main length axis of the body portions.

In the known Goniometer Pro application for smart phone, the user straps the smart phone to their limb, for example their forearm and then exercises the limb, for example by moving their arm through a range of motions. The angle of the smart phone relative to horizontal or vertical is measured using an in-built accelerometer in the smart phone.

In the case of the electronic goniometer applications for use with smart phones or other handheld devices, there are measurement errors due to misalignment of the main length of the smart phone screen or central axis of the smart phone screen with the main length of a body portion, as well as accuracy limitations from the orientation reading provided by the inbuilt sensors of the handheld computing device which rely on accelerometers. The accelerometers need to be referenced to a nominal vertical orientation and determine the orientation of the handheld device by measuring acceleration and deceleration as the handheld computing device is rotated or tilted relative to the initial reference position.

The known electronic goniometer smart phone applications require a setup procedure in which the smart phone is held level or upright, and requires a user to learn how to perform the setup procedure. The user has to set up the smart phone horizontally or upright so that the internal digital electronic spirit level in the device can record a reference attitude.

With the known electronic goniometers, the medical practitioner needs to be physically present with the person who is being measured so that they can perform the setup procedure and the measurements. Alternatively, if the subject of the measurements uses the known electronic goniometer remotely from the medical practitioner, they would need to be trained how to use it.

WO 2019/183733 A1 discloses a method and system for motion capture for use in enhancing human performance in an activity in military, entertainment, sports, and medical applications and comprises a camera system having one or more cameras and a depth sensor, and is capable of identifying a plurality of point coordinates corresponding to anatomical features of a human body. The system disclosed in WO 2019/183733 A1 captures user movements and matches them to one of more reference user movements, but cannot be used as a goniometer or to take goniometer angle measurements and does not have a protractor display. Another prior art method is known from US 2012/249416 A1.

### Summary of the Invention

According to a first aspect there of the present invention, there is provided a method of making goniometer angle measurements between anatomical parts of a human subject by processing data describing anatomical features of said subject;
said data comprising a plurality point coordinates in a first three dimensional coordinate space, said plurality of point coordinates representing anatomical features of said subject;
said method comprising:
   determining one or more geometric parameters of said plurality of point coordinates;
   grouping said plurality of point coordinates into groups of points each of which represents a said anatomical feature;
   recognising a said anatomical feature from said determined geometric parameters;
   comparing said determined geometric parameters with a set of predetermined geometric parameters stored in a database, each said set of predetermined geometric parameters corresponding to a respective pre-stored movement type for said recognised anatomical feature stored in said database;
   depending on a result of said comparison, identifying a movement of said plurality of point coordinates with a said pre - stored movement type;
   generating a measurement of an angle of said recognised anatomical feature; and
   generating, on a user interface, a real time protractor display for displaying an angle between at least two said groups of point coordinates.

Preferably the method further comprises comparing a posture of said subject, by comparing an orientation of a first set of three-dimensional coordinates representing an anatomical feature with a stored reference orientation for said anatomical feature; and
identifying whether said set of three-dimensional coordinates have an orientation which is outside a predetermined range of orientations relative to said reference orientation.

Preferably the method further comprises capturing a video image of said subject in said three-dimensional coordinate space;
generating a protractor which moves in real time corresponding with a movement of said subject; and
overlaying said protractor on said video image.

Said method may further comprise determining angular measurements between individual said groups of points each said group representing a said anatomical feature.

Said method may further comprise determining angular measurements between a first group of points representing a first said anatomical feature, and the second said group of points representing a second said anatomical feature.

Said process of determining one or more geometric parameters of said plurality of point coordinates may comprise determining parameters selected from the set:
an angle between pairs of point coordinates;
a line coinciding with a said group of point coordinates;
an angle between two different said groups of point coordinates;
a relative motion between two different said groups of point coordinates;
a speed of movement of individual point coordinates within 3D coordinate space;
a speed of movement of said groups of point coordinates in 3D coordinate space;
a relative speed of movement between individual said groups coordinates;
a direction of a line coinciding with a said group of point coordinates;
an orientation of a said group of point coordinates in 3D coordinate space;
a direction of rotation of a said group of point coordinates in 3D coordinate space;
a speed of rotation of a said group of point coordinates in 3D coordinate space;
a translation motion of an individual point in 3D coordinate space;
a translation motion of a said group of point coordinates in 3D coordinate space.

Said method may further comprise transforming a plurality of three-dimensional coordinates in a first coordinate space to a corresponding plurality of point coordinates in a second coordinate space.

Said point coordinates may represent anatomical features selected from the set:
skeletal joints of said subject;
individual limbs of said subject;
individual limb portions of said subject;
individual bones of said subject;
individual bone combinations of said subject.

The method preferably further comprises generating a real time protractor display for displaying an angle between at least two groups of point coordinates.

The method may further comprise:
comparing an orientation of a first set of three-dimensional coordinates representing an anatomical feature with a stored reference orientation for said anatomical feature; and
identifying whether said set of three-dimensional coordinates have an orientation which is outside a predetermined range of orientations relative to said reference orientation.

Other aspects are as set out in the claims herein.

### Brief Description of the Drawings

For a better understanding of the invention and to show how the same may be carried into effect, there will now be described by way of example only, specific embodiments, methods and processes according to the present invention with reference to the accompanying drawings in which:
Figure 1 herein illustrates schematically a known mechanical medical goniometer for measuring articulation of joints;
Figure 2 herein illustrates schematically use of a known mechanical goniometer for measuring an angle of extension of a human elbow joint;
Figure 3 herein illustrates schematically use of a known mechanical goniometer for measuring an angle of flexion of a human elbow joint:
Figure 4 herein illustrates schematically a known digital goniometer, providing a digital readout of angular measurement of articulated joints;
Figure 5 illustrates schematically a screen view of the known RateFast goniometer application which is based on a digital computing platform, such as a smart phone;

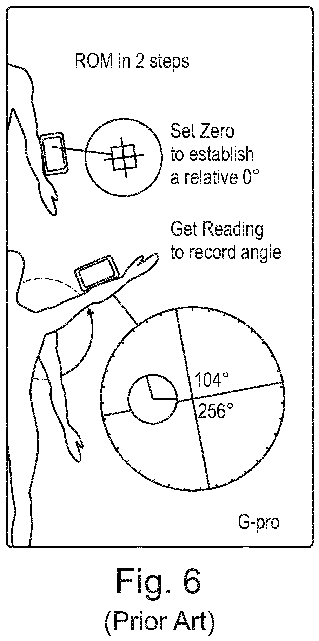

Figure 6 herein illustrates schematically a screen view of the known Goniometer pro (G-pro) device based upon a smart phone;
Figure 7 herein illustrates schematically in perspective view use of a motion assessment apparatus according to a specific embodiment and method according to the present invention;
Figure 8 herein illustrates schematically hardware components of an apparatus for measuring body movement and body flexibility;
Figure 9 herein illustrates schematically a structure of different processes and modules according to a specific embodiment;
Figure 10 herein illustrates schematically an example of processes carried out during an assessment session in a specific method according to the present invention;
Figure 11 herein illustrates schematically an example of processes carried out during an assessment session in a specific method according to the present invention;
Figure 12 herein illustrates schematically a view showing a two-dimensional screen image of a subject present in a first coordinate space, with a two-dimensional view of a plurality of 3 dimensional points in a second coordinate space, the three-dimensional points being shown as dots superimposed onto the two-dimensional image of the subject;
Figure 13 herein illustrates schematically a view showing a two-dimensional screen image of a subject in a first 3D coordinate space, having superimposed thereon a protractor display generated by a specific method according to the present invention;
Figure 14 illustrates schematically a screen view showing part of an assessment session comprising a two-dimensional image of a subject in a first 3D coordinate space having associated therewith a protractor display; a plurality of metric views each showing measured movement parameters, for an identified movement or exercise type, generated by a specific method according to the present invention;
Figure 15 herein illustrates schematically a screen view of a part of an assessment session in which a subject is incorrectly performing a particular identified type of movement or exercise, showing a two-dimensional image of a subject in a 3D first coordinate space accompanied by a graduated protractor, a gauge or dial type display showing spinal alignment and a written instruction to a user displayed on screen;
Figure 16 herein illustrates schematically a screen view of part of assessment session in which a subject is incorrectly performing a particular identified type of movement or exercise, showing a two-dimensional image of the subject in a 3D first coordinate space accompanied by an upright protractor and a lateral protractor superimposed on the image of the subject in a Main View together with a dial or speedometer type display showing a parameter of the subject which is outside a predetermined goal range for that parameter for the type of identified movement or exercise being carried out;
Figure 17 herein illustrates schematically a screen view of part of an assessment session showing in a Main View (first screen area) a two-dimensional video image of a subject in a first 3D coordinate space, and in a side view (second screen area) a 3D digitally generated dynamically moving anatomical view of internal anatomical features showing individual muscles and a part skeleton which are being assessed in the assessment session, and which anatomical view moves in synchronization with a subject's body movements;
Figure 18 herein illustrates schematically one example of an assessment report generated substantially in real time during an assessment session using a specific method according to the present invention, relating to a shoulder abduction exercise; and
Figure 19 herein illustrates schematically a second example of an assessment report for the same assessment session as represented by the report of Figure 18 herein.

### Detailed Description of the Embodiments

There will now be described by way of example a specific mode contemplated by the inventors. In the following description numerous specific details are set forth in order to provide a thorough understanding. It will be apparent however, to one skilled in the art, that the present invention may be practiced without limitation to these specific details. In other instances, well known methods and structures have not been described in detail so as not to unnecessarily obscure the description.

In the following description, the term "user" is used to denote the person operating the specific embodiment device or method as presented herein.

In this specification, the term "subject" is used to denote an object, primarily a human person, but which could also include an animal or robot, whose range of movement is being measured or determined.

In a situation where the subject is the human same person as the user, that is, the subject is taking their own measurements using the embodiments described herein, the subject is referred to as the user. That is, the user is taking measurements of themselves as subject.

In this specification, the term "posture" when referring to a subject is used to denote the way in which subject holds their body parts, for example, shoulders, neck, back, in relation to each other, and/ or to a particular attitude in which someone stands, sits, or lies down. Examples of posture comprise: upright, slouched; reclining; splayed; head drooping; relaxed; tense. The term "posture" may be used to describe various positions, and in general a posture is a type of position.

In this specification the term "position" in the context of referring to the position of a subject's body refers to an overall orientation of the body and /or a configuration of the parts of the body relative to each other. Examples of such positions include but are not limited to standing, seating, kneeling on one or both knees, lying down, reclining, a prone lying position, a supine lying position, raising one arm vertically, raising both arms, either vertically or at an incline to horizontal, holding one or both arms out substantially horizontally, raising one knee, or raising one leg. Position also applies to individual body parts, for example an arm can be in a straight position or a bent / angled position; a leg can be in a bent or extended position, and can be in an open position fingers splayed, and open position fingers together, or a closed position (fist). One position may have several alternative postures. For example a person in a seated position may be in an attentive upright posture, or in a slouched posture.

In this specification, modules named with the prefix AR are part of the known augmented reality kit of Apple Corporation^{®}. All other modules, classes routines and processes unless otherwise stated are created by the inventors herein.

### Overview

Referring to Figure 7 herein, there is illustrated schematically in perspective view a motion assessment apparatus 7000 according to a specific embodiment. The motion assessment apparatus comprises a hand held or human portable computing platform having an outer casing; a digital processor; and internal battery pack; a memory device; a data storage device; a video camera device, which may also capture still images; a light detection and ranging (LiDAR) device; an orientation measuring device to determine the orientation of the device, for example one or more motion sensors and/or accelerometers; and a user interface comprising a touch sensitive screen which can accept commands and instructions by a user touching the screen, the touch sensitive screen also comprising a visual display device which displays in real time, and image generated by the video camera device, and which can superimpose computer generated images onto a still or video image or scene displayed on the screen.

A human subject 7001 is positioned within a field of view of the camera device and the field of view of the LiDAR device of the motion assessment apparatus 7000. A real-time video image is captured by the camera device and converted to an image comprising a plurality of two-dimensional frames of pixels, as is known in the art. The video image comprises a plurality of a sequence of individual two-dimensional frames of image data captured at a predetermined rate of frames per second.

The LiDAR detector instrument itself is already known in the art and comprises a light pulse emitter which emits a sequence of light pulses, and a light pulse detector which detects reflected light pulses. The LiDAR apparatus shines an array of rapid pulses of laser light over a field of view, in this case, the first coordinate space within which the human subject is positioned, typically at over 150,000 pulses per second. A sensor detects a reflection of the light pulses reflected from the surface of the subject in the field of view and measures the amount of time which each pulse takes to be reflected from the pulse emitter. The round trip time from the time of emission of the pulse to the time of detection of the reflection of the pulse is proportional to the distance of the reflecting surface away from the emitter and sensor, and therefore by measuring the difference in time between emission of the pulse and a measurement of the distance of the reflecting surface in the field of view can be determined. The light beam is scanned side to side in a 3D array of beams, so that the direction / angle of the centre of the pulsed light beam is known relative to the main central axis of the LiDAR instrument, and provided the LiDAR instrument is held relatively stationary, the instrument can determine the position of a three-dimensional point within a first coordinate space from a calculation of the vertical and horizontal angle relative to the centre line of the LiDAR instrument and the time taken to reflect an emitted pulse, which gives the distance from the LiDAR instrument.

A human user 7002 holds the motion assessment apparatus 7000, pointing the camera and LiDAR instrument at the human subject in the field of view in the first coordinate space. The camera records frames of image data (video data) and the LiDAR instrument records frames of LiDAR data. The frames of LiDAR data comprise a plurality of three-dimensional coordinates each of which corresponds with a reflection point on the surface of the human subject, or any other object within the field of view, which has reflected light from the LiDAR light pulse emitter. Each LiDAR data frame therefore comprises a plurality of sampling points on the surface of a human subject, as well as reflected data points from other objects within the first coordinate space, for example walls, floor and ceiling.

The LiDAR camera captures data relating to the subject in a first 3D coordinate space, being the physical place or room where the subject is standing as a series of 3D coordinates. There is pre-processing into a set of dots which represent main anatomical components of the subject, which are processed at 60 frames per second. Subsequently those dots will be converted to a second 3D coordinate space which is more convenient for subsequent processing. The first 3D coordinate space is the 3D coordinate space corresponding to the real scene where the subject is standing and of which the video image and LiDAR data is captured, and the second 3D coordinate space is a virtual coordinate space used for further processing and analysis of the dots.

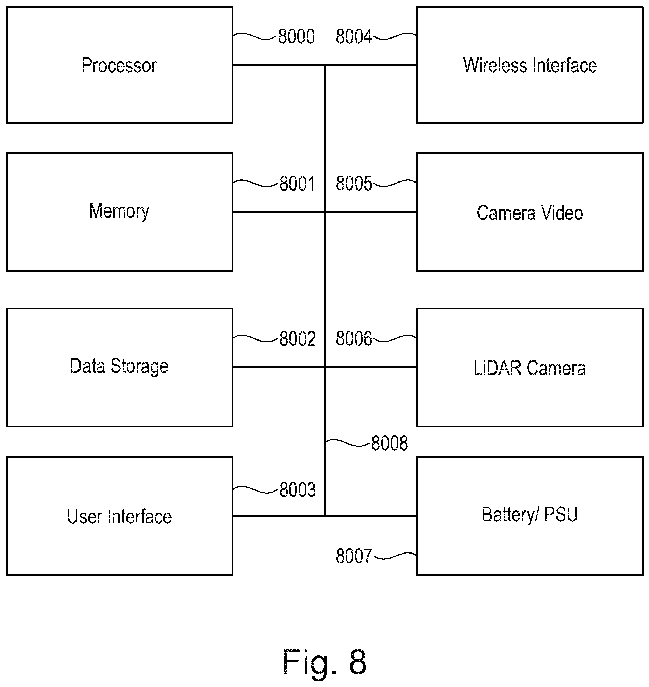

Referring to Figure 8 herein, there is illustrated schematically components of a user-portable computing platform comprising the apparatus for measuring body movement. The apparatus comprises a processor 8000; memory 8001; and electronic data storage device 8002; a user interface 8003 including a touch sensitive screen for receiving commands and instructions, and also for displaying images including video and still images; a wireless interface 8004 comprising a Bluetooth^{®} interface and a wifi interface; a digital video camera 8005, which is also capable of capturing still images; a LiDAR camera 8006 capable of capturing LiDAR images including depth / distance information; a battery and power supply unit 8007; and a data bus 8008 enabling all components to communicate with each other. The apparatus also comprises an outer casing. The user interface may comprise a keypad, which may be a touch screen keypad; a touch pad or mouse; various input and output ports; various physical connections, for example USB connectors; and one more ports for accepting a memory card, for example an SD or micro SD memory card. The user interface 8003 preferably also comprises a sound interface comprising an audio speaker, microphone, and a mic/ phono socket.

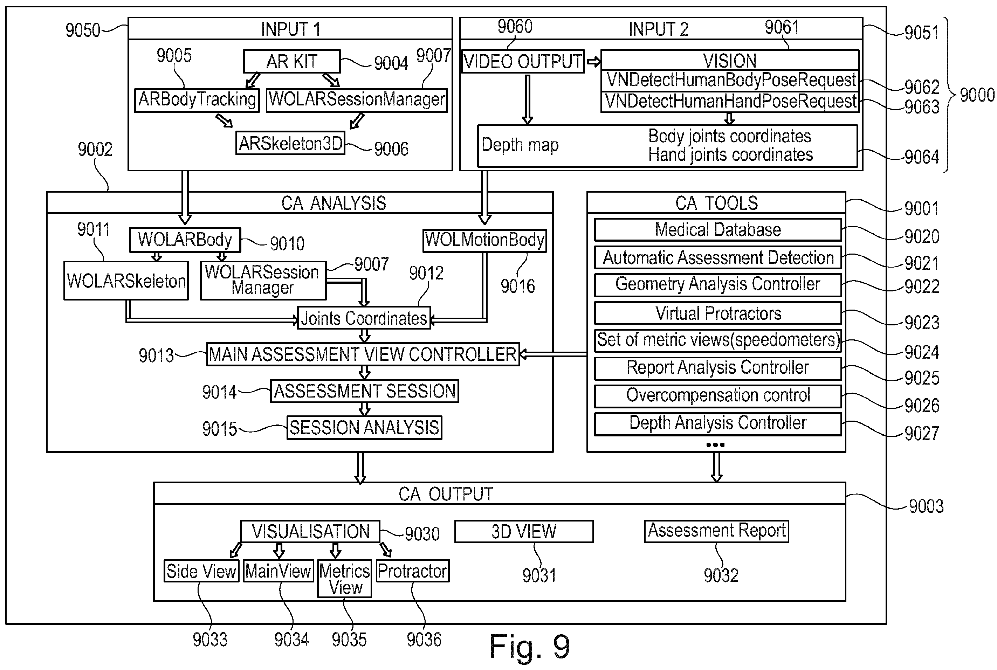

Referring to Figure 9 herein there is illustrated schematically an apparatus for measuring body movement and body flexibility.

The apparatus comprises a set of modules for carrying out the specific method herein, according to a first specific embodiment. In broad overview, the modules comprise an Input module 9000; a toolset module CA Tools 9001; an analysis module CA Analysis 9002; and an output module CA Output 9003. All modules operate processes in parallel to each other and communication between modules occurs in parallel unless otherwise stated.

The input module 9000 comprises a first input module 9050 and a second input module 9051. The first input module comprises Augmented Reality Kit ARKit 9004, which is an Apple^{®} product; ARBody Tracking 9005, also an Apple product; ARSkeleton 3D 9006; and WOLARSessionManager 9007. In Figure 8, all classes which begin AR are Apple classes, and all other classes are according to the specific embodiments and methods herein.

The second input module 9051 comprises a video output 9060; Vision 9061 which includes VNDetectHumanBodyPoseRequest 9062 and VNDetectHumanHandPoseRequest 9063; and Depth Map 9064, which includes Body Joints Coordinates and Hand Joints Coordinates.

CA Tools module 9001, comprises a medical database 9020; and automatic assessment detection module 9021; a geometry analysis controller 9022; a set of virtual protractors 9023; a set of metric views or speedometers 9024; a Report Analysis Controller 9025; and an Overcompensation Controller 9026; and Depth Analysis Controller 9027.

The CA Analysis module 9002 comprises WOLARBody module 9010; Joints Coordinates 9012; Main Assessment View Controller 9013, which runs an Assessment Session 9014 and provides Session Analysis 9015; and WOLMotionBody module 9016.

The CA Output module 9003 comprises visualisation 9030; 3D view 9031; and Assessment Report 9032. Visualisation 9030 comprises Side View 9033; Main View 9034; Metrics View 9035; and Protracter View 9036.

### Input Module

### ARKit module

The ARKit module is an Apple module which provides pre-processing of LiDAR and video data. Part of the ARKit is the ARBodyTracking function and the ARSkeleton3D module.

### ARBodyTracking module

When ARKit identifies a person in the back camera feed, generates an ARBodyAnchor which can be used to track the body's movement. Plane detection and image detection are enabled. If a body anchor is used to display a virtual character, the character can be set on a surface or image which is chosen.

ARConfiguration.FrameSemantics type bodyDetection is enabled by default, which gives access to the joint positions of a person that ARKit detects in the camera feed via the frame's detectedBody.

Within the ARBodyTracking module there is also the ability to track a person in the physical environment and visualizes their motion by applying the same body movements to a virtual character.

### ARSkeleton3D module

An ARBodyAnchor contains one instance of the ARSkeleton subclass to provide its joint positions in 3D space. The jointLocalTransforms property describes a joint's 3D offset from its parent joint. The jointModelTransforms property describes a joint's 3D offset from the body anchor's transform. The ARSkeleton3D input comprises instances of three-dimensional coordinate points on a body, for example a human body derived from the LiDAR data. ARSkeleton3D module supplies a continuous stream of data instances to start a session. Each ARSkeleton3D data instance comprises a set of coordinates in three-dimensional space derived from the LiDAR light ranging camera. The three-dimensional coordinates represent points on a subject image, in particular a human body. For example, a set of points in three-dimensional space coordinates is provided corresponding to a subject image of a human body where the 3D points represent individual joints located at or on:
- a centre line / mid line of the human body;
- a centre line of each arm of the human body;
- a centre line of each leg of the human body;
- a centre line of the head of the human body.

As a collection of joints, the ARSkeleton3D protocol describes the state of a human body whose movements ARKit can track. The ARSkeleton3D subclass provides the position of a tracked body's joints in 3D space, specifically with its jointLocalTransforms and jointModelTransforms properties.

Since the laser light beams do not penetrate the human body, and therefore cannot directly measure the position of the bones within the body, the ARSkeleton3D three-dimensional coordinates represent approximations of the positions of parts of main skeletal components of the subject human in the field of view of the camera and LiDAR sensor. For example, a human arm may be represented as a line of 3D coordinates in the first coordinate space in which the human subject is located.

The stream of data instances received from ARSkeleton3D is a real-time data stream, and comprises 60 instances per second, equivalent to a frame of data points, so that as a human subject in the field of view of the camera moves around, ARSkeleton3D provides a continuously updated dataset in real time representing 3 dimensional data coordinates of parts of the moving human body of the subject. The data received from ARSkeleton3D forms the input data stream to the CA Analysis module and CA Tools module. Typically each instance (frame) comprises around 93 individual three dimensional coordinates.

### WOLARSessionManager Module

The WOLARSessionManager module 9007 coordinates communication between the ARKit and ARSkeleton3D modules and the remainder of the system being the CAAnalysis module 9002, CA Tools module 9001 and CA Output module 9003. The WOLARSessionManager module also manages interfacing and communications between the CA Analysis module, CA Tools module and CA Output module.

### CA Analysis Module

The WOLARBody module 9010 in the CA Analysis module 9002 receives the datasets from the input module 9000 and creates a new set of instances of three dimensional coordinates from the ARSkeleton3D dataset, where the ARSkeleton3D dataset comprises a plurality of instances of three dimensional coordinates as described herein above. The dataset created by WOLARBody comprises an optimised and normalised version of the ARSkeleton3D dataset in a format which is optimised for use with the WOLARSkeleton and WOLAR SessionManager modules. The normalisation comprises creating a predefined three-dimensional space within the WOLARBody module, and fitting the ARSkeleton3D dataset within the predefined three dimensional space. To convert between ARSkeleton3D space and WOLARBody three dimensional space, each 3D point of each instance in ARSkeleton3D space is multiplied by a multiplication factor which fits the 3D point to a corresponding point in WOLARBody three-dimensional space.

The WOLARBody module 9010 produces a WOLARSkeleton model 9011 which comprises the normalised dataset output from the WOLARBody module. The normalised dataset comprises a plurality of individual instances, each instance comprising a plurality of individual three dimensional coordinates within WOLARBody space, the collection of three-dimensional coordinates representing coordinate positions on a human skeleton of the subject, in time. The WOLARSkeleton dataset comprises a plurality of instances, which represents coordinate positions of points of the human skeleton of the subject changing over time (at 60 frames per second in the example herein, each frame being a separate instance of the WOLARSkeleton dataset).

The continuous real-time data output stream of WOLARSkeleton provides a plurality of joint coordinates 9012. The joint coordinates are input into the main assessment view controller 9013.

The Main Assessment View Controller 9013 controls the view seen by the user on the user display screen. All of the tools in the CA Tools module 9001 are used by or are available for use by the main assessment view controller 9013.

The main assessment view controller may automatically start a session as soon as a recognisable movement is automatically detected by the Automatic Assessment Detection module 9021, and the Report Analysis Controller 9025 starts recording the session, and the Report Analysis Controller provides analysis and results of the movements of the subject during the session. Recording of session data and analysis of data and generation of reports continues in parallel. Alternatively a session may be started by manually activating a session by a user.

### CA Tools Module

Medical Database 9020 contains data describing a plurality of different human movement and position types. Each different movement type and each position type is stored as a record comprising a set of three-dimensional coordinates and including parameters such as angle between sets of three-dimensional coordinates which are characteristic of a particular posture or movement.

The types of body exercises involving adopting movements, positions may include any one or more of the following examples: standing upright with arms hanging vertically downwards; a sideways rotational stretch; and arm raised(left arm); and arm raised (right arm); arms raised, both right and left arms; a forward standing bend; a backwards/lean back; a head rotation left; a head rotation right; a head tilt left; a head tilt right; head raise; head lower; an upper torso twist left; an upper torso twist to the right; a leg extension/leg bend movement (left leg), leg raise to the front (left leg); leg raise to the front (right leg); leg raise to the rear (left leg); leg raise to the rear (right leg); leg raise to the side (left leg); leg raise to the side (right leg); a leg extension/leg bend movement (right leg); an elbow extension/elbow bend movement (left arm); an elbow extension/elbow bend movement (right arm); a wrist bend movement (left wrist); a wrist bend movement (right wrist); individual extension and bend movements for each finger and thumb of each hand; flexion of ankle, left or right; movements performed whilst sitting or lying down. In principle, any movement, position or posture of any part of or of the whole of the human body may be included in an exercise which is monitored, measured and/ or analysed by the apparatus herein.

For each individual position, posture or movement record in the medical database the following information may be stored: name of the movement; target range of the movement, for example angular range in degrees; the primary and assistant muscles used in the movement; articulation name, myotome, antagonistic motion. Further like information may be saved to the medical database.

The Automatic Assessment Detection module 9021 identifies which type of movement or posture a subject is adopting from the real time stream of Joint Coordinates and matches that movement or posture type to an equivalent pre-stored movement or posture type in the medical database. Where the Medical Database does not contain an equivalent pre-stored record for a movement or posture type equivalent to a movement or posture which a user is adopting, optionally the automatic assessment detection module may be configured to create a new database record corresponding to the new movement or posture.

Using the medical database, the automatic assessment detection automatically identifies which type of movement the subject is performing. It does this by using a series of geometry calculations which are calculated in the Geometry Analysis Controller 9022.

Using the Medical database 9020, the automatic assessment detector 9021 automatically identifies which movement a subject is performing by comparing the joint coordinates 9012, output from the WOLARBody, which in turn are derived from the continuous stream of LiDAR derived three dimensional points output from ARSkeleton3D, with the records stored in the medical database 9020 to determine which, if any of the records in the medical database matches the movement or posture represented by the set of joint coordinates 9012 output from WOLARSkeleton module 9011.

Medical Database 9020 may be implemented as a plurality of data records, for example in spreadsheet form wherein on one axis are listed all of the different possible types of exercise which are subject may carry out, and on another axis, all of the different parameters relating to each individual type of exercise, the parameters defining the thresholds for what constitutes what is an acceptable or correct movement of that type, including maximum and minimum angles of movement between sets of joint coordinates, where sets of joints are grouped to represent individual body features such as joints, limbs, digits (fingers, toes) or the like. The Medical Database stores records comprising the following information types:
- a type of movement or exercise (name of movement);
- a type of posture (name of posture); and

For each type of movement and/or posture:
- a set of points corresponding to one more individual body parts;
- which individual body parts need to be tracked / monitored for the particular type of movement;
- which individual joints need to be tracked/monitored for the particular type of movement;
- a range of maximum angles and minimum angles corresponding to angles between individual body parts, which are correct or acceptable for the particular type of movement or posture, including threshold values for those parameters;
- a range of maximum angles of a main length of a particular body part compared to a particular coordinate in coordinate space, which is in within limits for the type of movement, including threshold values for those parameters;
- which combination of the above parameters are included for each individual movement or each individual posture.
- a corresponding 3D motion animation

By comparing a combination of parameters, for example horizontal movement plus circular movement, determined by the Geometry Analysis Controller 9022, against a combination of parameters stored against each type of movement or each posture in medical database 9020, then the individual movement or posture type can be identified from the medical database.

Having identified a particular movement type or posture type from the medical database which corresponds with the data being generated from the geometry analysis controller, the individual upper and lower limit parameters for that identified movement or posture can be read from the pre-stored record of that movement type from the Medical Database 9020. This information is used by Over Compensation Controller 9026 to confirm whether the subject is performing the movement correctly or not.

For example, during a shoulder flexion, the spine needs to be kept upright within limits whilst the shoulder is being flexed. The data record for that type of movement in the Medical Database 9020 may state that for this exercise, the shoulder angle and the spine angle need to be monitored and are also displayed on the user interface in real time displays of virtual protractors, one for each angle.

Over compensation controller 9026 continually monitors the necessary parameters as read from medical database 9024 during a shoulder flexion movement by the subject, and using virtual protractors 9023 which output an angle reading of the angle of the arm of the subject in real-time during a shoulder flexion exercise, and by monitoring information from the Geometry Analysis Controller 9022 describing the attitude of the spine of the subject in real-time, the Over Compensation Controller 9026 can monitor that the subject spine is being held substantially vertical, within angular limits specified by the appropriate record in the medical database 9024 that shoulder flexion exercise, and therefore determined that the exercise is being carried out correctly.

However, if the over compensation controller 9026 determines that the geometry analysis controller is outputting a signal for the attitude of the line of the spine of the subject which is too far away from vertical, outside of the limits contained in the appropriate record in the Medical Database 9020, then the Overcompensation Controller 9026 will generate an alert signal.

By comparing the actual angles of movement of body features of the subject with the corresponding acceptable angle stored in the medical database it can be determined whether the movement is being carried out correctly by the subject without the need for a medically trained human movement is being carried out correctly.

As an example, if the subject is lifting their arm up in front of them, the medical database will contain a record containing information of which joints of the body need to be tracked, and what is a safe or correct maximum and minimum angle of movement of the arm within which the subject is deemed to be performing that movement or exercise correctly.

The Automatic Assessment Detection module 9021 gets data from the Geometry Analysis Controller 9022 and from the medical database 9020, and compares those two sets of data to match the Geometry analysis data with equivalent data from a pre- stored record in the medical database. Provided the angles in the geometry analysis data are within limits, the same as the angles for a record in the medical database, corresponding to a particular movement type, then the Automatic Assessment Detection Module 9021 recognises the geometry analysis data as being the same type of movement which corresponds to the record containing the similar angle data in the medical database.

The Geometry Analysis Controller 9022 carries out a series of geometric calculations by examining every set of three-dimensional points comprising the joint coordinates 9012, to provide angle data and other metrics, for example rate of change of angle, and change of direction of lines of 3D points, rate of change of direction of lines, rate of change of absolute position of individual 3D points and like parameters describing the absolute positions in 3D coordinate space of the joint coordinates, and describing the relative positions of individual joint coordinates relative to each other. For example, the change in absolute position of a joint coordinates in 3D coordinate space may be determined by the geometry analysis controller, for example, corresponding to an absolute distance shift in movement of an elbow joint in the first coordinate system which the subject occupies. The Geometry Analysis Controller 9022 and controls every operation carried out on the joint coordinates 9012.

The geometry analysis controller 9022 provides a tracking function which tracks the movement of a subject's body parts in real time.

The data input to the geometry analysis controller comprises numerical data corresponding to a plurality of dots as viewed on the user screen, each dot representing a three-dimensional point in three-dimensional coordinate space, wherein the dots move in real time following real-time movements of the subject and approximate positions of points on or within the body of the subject. The input dots comprise three-dimensional real-time moving data points in 3D coordinate space which are apparently unrelated to each other in the first instance, except in the sense that all dots will relate to a position on, in or immediately adjacent the subject. Creation of further relationships between the dots in order to group the dots into sets of dots each of which represents a body part or other body feature is carried out in the geometry analysis controller. The input dots need to be analysed in order to group them into sets, each set corresponding to and representing an anatomical feature of the subject.

Such analysis may comprise applying correlation algorithms to pairs or groups of dots to determine for an individual dot whether the movement of that dot correlates closely or not with any other individual dot, and thereby to determine which plurality of dots share strong correlations of movement with each other and therefore can be assigned to a common named body part, and which individual dots have little or no correlation with other dots, from which it can be determined that those dots do not belong to a same named body part.

Angles between sets of 3D coordinates of the Joint Coordinates are calculated in real time and continuously updated. The output of the geometry analysis controller comprises angle data describing angles between individual sets or lines of 3D coordinates.

The output of the geometry analysis controller comprises the following data:
- Positions of dots within 3D coordinate space (second coordinate space);
- groupings of dots into sets representing three-dimensional structures (body features of the subject);
- beams or lines, including direction in the 3D coordinate space, and length of beam or line;
- angles between groups of dots, corresponding to angles between three-dimensional structures or body features of the subject;
- speed of movement of individual dots in the second coordinate space;
- speed of movement of groups of dots in the second coordinate space;
- acceleration / deceleration of dots moving in the second coordinate space
- direction of movement of dots within the second coordinate space

All of the above data outputs are derived from or determined from the plurality of 3D joint coordinates, i.e. the plurality of dots which are input into the geometry analysis controller, which are derived from the original ARSkeleton3D data.

Combinations of output parameters of the Geometry Analysis Controller are recognised by comparison with the movement type records stored in the medical database 9020. For example if a group of 3D points is recognised as forming a particular pattern in second coordinate space, and/or having a particular angle or orientation within the second coordinate space, and that pattern matches a corresponding pattern of a body part record stored in the Medical Database, then that pattern of dots may be recognized as being the corresponding body part.

The Geometric Analysis Controller also provides geometric data, for example angle data to the Set of Metric Views module and the Virtual Protractors.

Virtual Protractors module 9023 generates data describing a set of virtual protractors which are displayed adjacent to an image of the human subject on the visual display device. The virtual protractors may overlay the image of the human subject as presented on the screen, or may lie adjacent to the image of the human subject. The virtual protractors show which angle is being measured, for example the angle between the main line of an arm and the mainline of a torso, and display a numerical value for that angle in real time. The virtual protractors may be overlaid onto a two-dimensional image of the human subject on the visual display device.

The virtual protractors module 9036 is responsible for drawing protractors on the screen of the user interface. The virtual protractors module is driven by the geometry analysis controller 9022 and by the automatic assessment detection module 9021.

A Metric Views module 9035 generates metric views, for example a speedometer view which displays in real time a rate of change of angle of a particular skeletal component for example an arm, relative to another skeletal component, for example a spine. A metric view typically comprises a numerical display which changes in real time depending upon the parameter which it represents. For example a metric view may comprise a real-time display of angle in degrees, which changes value in real time when the angle being monitored changes in real time. For every movement, using the Medical Database 9020, there is specified a particular type of metric view corresponding to that type of movement, so that by automatically detecting the type of movement that subject is making using the Automatic Assessment Detection module 9021, the appropriate types of one or more metric views can be looked up in the Medical Database, and those one or more metric views can be generated and displayed on the screen of the visual display device, as controlled by the Main Assessment View Controller 9013.

Report Analysis Controller module 9025 records all of the joints coordinate data collected within a session in real time. Data is recorded within the session. All of the metrics and parameters which are viewable on the screen by the user stored by the Report Analysis Controller within the session.

In a further embodiment, the records of the joints coordinate data collected within a session and all other session parameters and data may be saved locally for sending to a remoter location or server after a session.

An Overcompensation Controller 9026 analyses the joint coordinates 9012 and identifies joint coordinates corresponding to particular skeletal components, for example a subject's spine, and also contains algorithms for comparing the overall posture or position of the subject with the pre-stored records for the appropriate movement type stored in medical database 9020. Each movement record in the medical database has joint coordinates within 3D space in the second coordinate system corresponding to the ideal or "within limits" orientations, positioning and attitudes of individual skeletal components which are adopted when a movement is being performed correctly.

Information from each of the medical database 9020, automatic assessment detection 9021, geometry analysis controller 9022, virtual protractors module 9034, the metric views 9024, Report Analysis Controller 9025 and Overcompensation Controller 9026 are fed into the main assessment view controller 9013 which controls the view on screen seen by the user.

For example when a subject stands upright and raises or lowers their arm, to obtain a goniometer measurement of minimum arm position (arm hanging vertically down from shoulder) and maximum arm position (e.g. arm raised to maximum extent), the subject should stand straight and upright, which means that as viewed from the front, their torso is substantially vertically upright, and the position of their spine, approximated as the centre line of their torso is also vertically upright.

The comparison of the subject's posture or movement with the reference posture or movements stored in the medical database 9020 can be made in various ways. In a first method, the geometry analysis controller 9022 identifies lines of joint coordinates corresponding to individual anatomical features, such as bones, and analyses the spatial relationships between the individual lines of joint coordinates, each line representing a different anatomical feature, to determine whether the main length axes of those anatomical features are within predetermined angles relative to each other.

For example, in the case of a subject arm, a first set of joint coordinates corresponding to a lateral epicondyle of humerus bone, a second set of joint coordinates corresponding to a radial styloid process, and the third set of coordinates corresponding to acromion, may each be identified automatically and adjusted manually. Additionally, there may be identified a fourth set of coordinates which correspond with other areas of the subject's body. Whilst the sets of 3D joint coordinates corresponding the respective lateral epicondyle of humerus, radial styloid process and acromion features are moving in real time relative to each other, at the same time there is identified a set of joint coordinates corresponding to the body, and in real time, the coordinates corresponding to the body are continuously monitored to see if they are substantially within the coordinate space, within a predetermined limit which is read from a record in the medical database. The record in the database may comprise an angle of deviation from the vertical (Z) coordinate, and to determine whether the subject's posture is outside of predetermined limits, a best fit straight line according to a mathematical equation, for example a least squares fit equation or the like is continuously calculated, from which a best fit line through the 3D coordinates corresponding to the subject's spine can be determined. The angle which the line makes to the vertical Z axis in coordinate space is continually monitored and compared to a corresponding angle parameter stored in the data record of the medical database corresponding to that particular type of movement. If the angle which the real time calculated line makes to the vertical Z direction deviates above the predetermined angular limit, this signifies that the subject is leaning over too much, in which case the overcompensation controller 9026 generates a signal signifying that the subject movement is outside parameters for the particular movement being performed. The overcompensation controller sends a signal to Report Analysis Controller 9025 which stops recording the stream of 3D joint coordinate data, until such time as the angle between the continuously recalculated line corresponding to the subject's spine is within a predetermined angular value compared to an upright vertical line in the coordinate space.

The Overcompensation Controller 9026 continuously compares the stream of frames of 3D joint coordinates 9012 corresponding to the spine of the subject, calculates whether those joint coordinates represent a vertical spine, or if not vertical, then calculates an angle from vertical in the second coordinate space, compares the angle of the joint coordinates representing a maximum allowed angle which is within limits for that type of movement, and which is stored as part of the medical record for that particular movement type.

If the movement of the subject's spine, as represented by the real-time data stream of joint coordinates is out of limit, that is, the angle of the spine to the vertical is outside the range of angles specified in the medical record for that particular movement type, then the Overcompensation Controller generates a signal which is sent to the CA Output module 9003 so that the Visualisation module can generate signals which indicate on the screen view that the movement is not being carried out correctly. This could be, for example a flashing protractor, or metric display, or a change of colour of the protractor or metric display.

### Protractors

Protractors are generated according to the virtual protractors module 9023 and are visualised by the protractor visualisation module 9036 which generates a user display for a protractor. A protractor comprises a linear scale with a graduation and a pointer. There may be provided a ribbon indicator. The linear scale may be circular or part circular, and the graduation is preferably denoted in degrees, but may be denoted in radians. The pointer may comprise a rotating needle type pointer which revolves around the linear scale to point at an angle on the graduation. The ribbon indicator preferably comprises a highlighted line extending in a circular path over an angle, where the angle represented is a target angle of movement.

The metrics which the pointer and the ribbon indicator are assigned to on an individual protractor display may be as follows. The pointer may be allocated to indicate the following metrics:
- current angle of movement of a body part or limb;
- Ideal or target angle of movement of a body part or limb (maximum extent of movement).

The ribbon indicator may be allocated to indicate any one of the following metrics:
- measured range of movement of a body part or limb during a session, with the ribbon display extending between a maximum and minimum angle;
- ideal range of movement of a body part or limb for a particular type of movement or exercise, with the ribbon display extending between a maximum and minimum angle.

### Overcompensation Alert Signals

When the overcompensation controller 9026 generates an overcompensation alert signal as a result of determining that a subject is performing an exercise with an incorrect posture or attitude, the overcompensation alert signal inhibits the Report Analysis Controller 9025 from recording data, until the overcompensation controller 9026 either ceases to generate an overcompensation alert signal, or generates a cancellation signal cancelling the overcompensation status. This means that the device does not record any movement data of the subject whilst the subject is in an incorrect posture for the type of movement or exercise being carried out.

Generation of an overcompensation alert signal generated in response to any particular parameter going outside predetermined limits, for example a shoulder angle going outside predetermined limit, is used to modify the protractor image output 9036, for that particular parameter, typically by turning the protractor display a different colour e.g. red, so that the user can see that the parameter is outside the limits set for performing the exercise correctly.

An overcompensation alert signal also changes the colour of reports, and hides odometer views on the screen.

### Grouping of Dots

The data input to the geometry analysis controller 9022 comprises a series of dots, the position of each of which in the second three dimensional coordinate space is updated at 60 frames per second. These dots are nominally uncorrelated to each other, that is to say there is no separate correlation data between the dots as they are input into the genre tree analysis controller. However, because the dots received from ARSkeleton3D represent locations on a subject's body there is some correlation between individual dots and groups of dots. The geometry analysis controller determines correlations between the dots and arranges the dots into groups of dots, where each group of dots represents a unique anatomical feature of the subject.

The Geometry Analysis Controller applies more algorithms which find correlations between individual pairs of dots and determines how closely collorated individual dots are with respect to other individual dots within the input stream of data in order to group dots together. The correlation functions include:
- comparison of instantaneous position of a dot with an instantaneous position of each other dot in the second coordinate space - dots which are immediately adjacent to each other are likely, but not necessarily, to be part of the same anatomical feature;
- comparison of speed of movement of a dot with a speed of movement of each other dot - dots which move at the same speed as each other, or one dot which moves at a speed within a threshold variation of the speed of movement of another dot is likely to be part of the same anatomical feature as that other dot;
- comparison of direction of movement of a dot with each other dot - two dots which are moving in the same direction as in three dimensional coordinate space are likely to be connected with the same anatomical feature;
- line drawing algorithms - by drawing lines connecting pairs of immediately neighbour adjacent dots, the directions of individual pairs of dots can be compared with the directions joining other individual pairs of dots. Series of three or more dots which form straight or near straight lines, are more likely to be connected with the same anatomical feature as each other;
- Rate of acceleration or deceleration - dots which are accelerating or decelerating at the same extent, or within predetermined ranges of variation as each other are more likely to be part of the same anatomical feature.

The geometry analysis controller uses one more of the above algorithms, to arrange the dots into groups of dots, where each group of dots is assigned to represent a particular anatomical feature. Preferably the anatomical feature is a body part which is formed around a particular bone between skeletal joints. For example, the upper arm formed around a humerus make be identified or defined as an anatomical feature; the forearm portion may be identified or defined as another and separate anatomical feature; and a joint region between the upper arm and forearm, corresponding to an elbow joint may be defined as an anatomical feature. Each anatomical feature is represented by a group of one more of the dots.

### Identification of Groups of Dots with a Subject's Anatomical Features

Whilst the geometry analysis controller forms groups of dots, each of which group represents a distinct anatomical feature, those anatomical features need to be recognised and identified in relation to the subject's anatomy. Recognition of anatomical features may be achieved by algorithms which examine the following parameters;
- number of inflections in a line of dots - by examining the number and sequencing of inflections of curvature of a line of dots, and by comparing that with pre stored data for patterns of dots to represent a particular anatomical feature, the anatomical feature can be identified using the inflections of a group of dots;
- absolute distance between dots in a group relative to the second coordinate system;
- angle of motion of a group of dots - by examining the angle of motion of a group of dots, the possible anatomical features which that group of dots can be determined by excluding anatomical features which are incapable of moving over the angular range of movement which a group of dots has moved in 3D coordinate space;
- speed of movement of a group of dots - by examining the speed of movement of the group of dots, then the range of possible anatomical features which that group of dots may represent may be reduced or narrowed. For example, fingers can be moved much more quickly than a complete arm. If the group of dots are moving at a speed greater than can be achieved by a human arm, then the anatomical feature of a human arm can be excluded from the possible set of anatomical features which the group of dots represent, whilst fingers remain within that possible set of anatomical features.

### Recognition of a Movement or Exercise

The recognition of a particular movement or exercise is carried out by algorithms in the Automatic Assessment Detection module 9021 based upon groups of dots output from the Geometry Analysis Controller 9022. The automatic assessment detection module uses data and information from the geometry analysis controller, which includes for each group of dots, parameters comprising: speed of movement of a group of dots; a nominal identification / naming of the group of dots, for example identifying the group of dots as a particular anatomical feature; an angle of movement (range of angular movement) of the group of dots; a shape of the group of dots; and orientation of the group of dots within the second 3D coordinate space.

For example, if the genre tree analysis controller outputs data for a particular group of dots, specifying that that group of dots is moving over a particular range of angles, and at a particular angular speed of movement, the automatic assessment detection module 9021 may compare that information with data stored in medical database 9020 with the result that it determines that that combination of speed and angular motion of the group of dots can only correspond with the predetermined possible movements of, for example a movement or exercise of raising and lowering a right arm, and therefore the automatic assessment detection module can identify that group of dots with the particular record for that movement or exercise stored in the medical database 9020.

### Report Analysis

Having identified which movement or exercise a subject is performing, from the automatic assessment detection module 9021, and the overcompensation controller 9026 is not generating an overcompensation alert signal, confirming that the subject is moving within the predetermined allowable range of parameters for a particular exercise, the Report Analysis Controller 9025 generates data to produce a graphical report in real-time which records movements carried out by the subject.

A visual representation of the report is generated by Assessment Report module 9032.

### CA Output

Visualisation component 9030 comprises 3D View visualisation component 9031; Assessment Report visualisation component 9032; Side View visualisation component 9033; Main View visualisation component 9034; Metrics View visualisation component 9035; and Protracter visualisation component 9036.

### 3D View

3D View visualisation component 9031 generates an animated screen display of muscle motion including skeletal components. The data for the 3D View is obtained from a known muscle motion animation generator. The type of one or muscles are identified automatically from the data record for particular identified movement or exercise stored in medical database 9020. The data describing the movement type is sent to the external muscle motion animator which has pre-stored 3D models of which particular muscles and skeletal groups are used for each identified movement type. The animation engine returns a rendered image comprising skeletal components together with a rendered image representation of the identified one or more muscles. Data describing the type of muscle, the angular orientation of the limb or other body part to which the muscle is attached is sent from the Main Assessment View Controller 9013 to the prior art muscle animation module, at 60 data updates per second, which returns a rendered image of a dissected anatomy of part of a virtual subject showing the relevant muscles which are movable in real-time according to the same instantaneous angles, orientations and attitudes adopted by the subject and as identified by the Geometry Analysis Controller 9022.

Having selected one or muscles, the muscle animator animates movements that those muscles make, for example when bending an elbow, or lifting a leg. To create the animation, the subject movement is identified by the automatic assessment detection module 9021 based upon information generated in real-time in the geometry analysis controller 9022, and referring to data in the medical database 9020 as hereinbefore described. Once it is known what the type of movement is, and the medical database has been checked to see if there is already a record for that type of movement in the medical database, and that there is a 3D model animation stored in the medical database for that type of movement, the relevant animation model is launched via WOLARSessionManager 9007 which acts as a bridge to the animation engine, and the animation engine returns rendered images which are converted into a screen display by 3D View component 9031.

In the 3D View display, when the subject bends their arm for example the Geometry Analysis Controller 9022 determines the orientations, angles, velocities of the groups of dots representing anatomical components of the arm, these are sent to the Main Assessment View Controller 9013 which manages sending and receiving data from the animation engine and displaying the animated computer-generated images on the side view of the screen, in real-time so that as the subject moves their arm in real-time, the animated anatomical display moves correspondingly in real-time.

### Sound Prompts

At various stages of operation, sounds may be played by a speaker or via a microphone socket of the user interface to give the user intuitive feedback during operation of the apparatus. Sound prompts may be generated at the following events:
- when an ARKit Anchor is added 1001;
- as the subject carries out a movement, for each 10° increment of a limb, there is an audio sound generated, for example a "click" sound;
- if the subject over compensates, an alert sound is generated;
- a sound is generated if a subject reaches a new personal best maximum value during a movement going

### Assessment Report

Assessment Report 9032 receives data from Report Analysis Controller module 9025 and Assessment Session 9014. Metric Views module 9024, Report Analysis Controller 9025, Overcompensation Controller 9026 and Assessment Session 9014 work together, each providing data input to the Assessment Report 9032. An individual assessment report output of the session may include the following data:
- maximum range of movement for the session;
- when a maximum range of movement was achieved during the session
- when a maximum range of movement was not achieved during the session;
- when the overcompensation limit was reached when trying to achieve a movement.

The above assessment report data is preferably displayed in graphical format.

### Method of Operation - Overview

The embodiments are intended for use with a user operating the measuring device, and directing the field of view of the measuring device at a subject. The measuring device may be able to be operated by the subject themselves if the device is placed on a tripod or other holding device, so that the subject may be the same person as the user, but primarily the measuring device is intended to be operated by a user who is different and separate from the subject being measured.

The user points the camera of the host handheld computer at the subject whose range of movement is to be determined. The subject resides within the field of view of the video camera and LiDAR camera within real space, which is the first coordinate space. The user reads information from the screen which instructs the subject how to stand or lie, and to how to perform a particular type of exercise or movement, for example to stand up straight and raise right arm, keeping the right arm straight. The user verbally relays these instructions to the subject, who carries out those instructions to the best of their ability.

Continuously throughout a session, an on-screen menu presents written instructions and information to the user to direct the user how to use the apparatus. The instruction set comprises:
- Instructions concerning the subject's posture or position, and how to correct the posture for example 'Lay your head flat against the ground';
- Instructions on moving individual anatomical parts of the subject, for carrying out an exercise or movement example 'Flex your elbow by bringing the palm of your hand towards your shoulder as much as each instruction depending upon the particular type of movement or exercise which is being assessed;
- Instructions and information on management and administration of a session, for example entering name, patient number, clinician name and the like. The instructions and information on management and administration may only be required if the session information is to be stored remotely after a session.

The measuring device may analyse the dots which align with the subject's body parts in various positions and can determine whether a subject is adopting a posture which is detrimental to the examined position, for example by not keeping their spine straight during a standing position exercise, or by slouching during a sitting exercise, and generate an alert message viewable by the user so that the user can verbally relay instructions to the subject so that they can correct their posture during an exercise.

ARSkeleton3D module supplies a continuous stream of data instances. When a stream of dots is received from ARSkeleton3D, those dots which are initially coordinates in first coordinate space are prepared by converting them to coordinates in second coordinate space by WOLARBody module 9010. WOLARBody is an instance of a class which in itself has information about the skeleton of the subject which is being measured, including height and other parameters. WOLARBody module 9010 forms a set of calculations on the ARSkeleton3D data to convert the dots from first coordinate space to the second coordinate space, in which all subsequent processing is performed. The initial coordinate data of dots from ARSkeleton3D which derive from the first coordinate space is normalized to fit into the second coordinate space by WOLARBody module 9010 and WOLARSkeleton module 9011. The second coordinate space is the three dimensional scene which is used for further processing, analysis and visualisation and corresponds with the view and scene as seen on the user display screen.

The class WOLARBody works in parallel with class WOLARSessionManager to project the original dots on to the three-dimensional scene of the second coordinate space and to produce Joint Coordinates 9012 in the second three-dimensional coordinate space.

The processing carried out by WOLARBody module 9010, WOLAR Skeleton module 9011, WOLARSession Manager 9007 to generate Joint coordinates 9012 could be described as pre-analysis to generate the Joint Coordinates data, which is pre-processed data derived from the dots supplied as a stream of coordinates in 3D first coordinate space. Joint Coordinates data comprises a set of dots presented as a dynamic stream of 3D coordinates in second 3D coordinate space.

The main analysis is managed by the Main Assessment View Controller 9013. As soon as Joint Coordinates data is available, then analysis by the CA Tools is performed.

As soon as Automatic Assessment Detection Module 9021 recognises a movement, then an Assessment Session starts. The Assessment Session module 9014 is a sub module of Main Assessment View Controller 9012. The Main Assessment View Controller manages Assessment Sessions, and if the user presses an on-screen RESET command, the current Assessment Session will be terminated, and the new Assessment Session may commence.

Assessment Session 9014 is an entity which stores information but has no actions. Session Analysis 9015 is an entity which does have actions, as described herein, including which parameters to analyse, which practice to draw, which speedometers to show, and how to report the data.

The CA Tools module 9001 automatically recognises which type of exercise the subject is performing by the Geometry Analysis Controller 9022 continuously in real-time analysing the movement of dots in second coordinate space, supplying that information to the Automatic Assessment Detection module 9021 which compares the data provided by the Geometry Analysis Controller with a plurality of records in the Medical Database 9020 until a match is found and the movements of the subject can be matched with a known pre-stored exercise or movement, for example shoulder flexion.

Optionally, the Input Module 9000 may further comprise a known Apple Create ML Action Classifier module. This module can be trained by entering ideally 50 or more video instances of a range of different persons performing the same physiotherapy exercise, for example arm extension and contraction (bending and elbow) into the known Apple Action Classifier module. After training, Apple Action Classifier on a training dataset, Action Classifier will then automatically thereafter recognise that movement or physiotherapy exercise type in new videos during an assessment or measurement session, and will return data identifying the type of physiotherapy exercise being carried out. That information can be used to automatically select the correct appropriate type of record in medical record database, i.e. pick a record according to the identified type of physiotherapy exercise or movement actually being carried out by the subject.

Once the type of movement which the subject is performing has been recognised and matched with a movement type in the medical database, the pre-stored parameters for that type of movement apply to the session. These pre-stored parameters comprise target / goal values for range or angle of movement of particular body parts which the subject should try to achieve, and a range of limits on posture variations, for example keeping the subject spine within predetermined limits of vertical within the second coordinate space, or relative position of the subject's head (e.g. keeping the head upright) which must be maintained for the movement or exercise to be performed correctly.

At all times during the session, Overcompensation Control module 9026, receiving data from Geometry Analysis Controller 9022 and Medical Database 9020 continually checks to see if any of the groups of dots representing body parts are out of limit for the movement being carried out and can interrupt data collection by the Report Analysis Controller 9025 during periods when the movement is out of limits.

Visualisation modules 9030 - 9036 operate continuously throughout the session to generate their respective views for real time display on screen.

The whole session is recorded by report analysis controller 9025, and so after movements have finished, and an assessment report is created, the session can be replayed on screen.

Outputs comprise visual outputs viewed on the user display screen. In the best mode, the views are separated into a side view, a main view, a metrics view and protractors.

The Side View 9033 comprises: the name of the movement; the goal range; the current range.

The Main View 9034 is the part of the screen where the video image of the subject patient is displayed, and all superimposed information drawn on top of all superimposed on the main view, for example a movable protractor, an image of the dots representing skeletal components or body parts.

Metrics View 9035, in the best mode is shown on the right-hand side of the screen and comprises a plurality of individual speedometer type displays in the form of circular dials with movable pointer needles.

Protractor View 9036 shows one more protractors which can be superimposed onto the video image of the subject, or which may lie adjacent the video image of the subject, within main view 9034.

### Specific Method for Conducting a Session

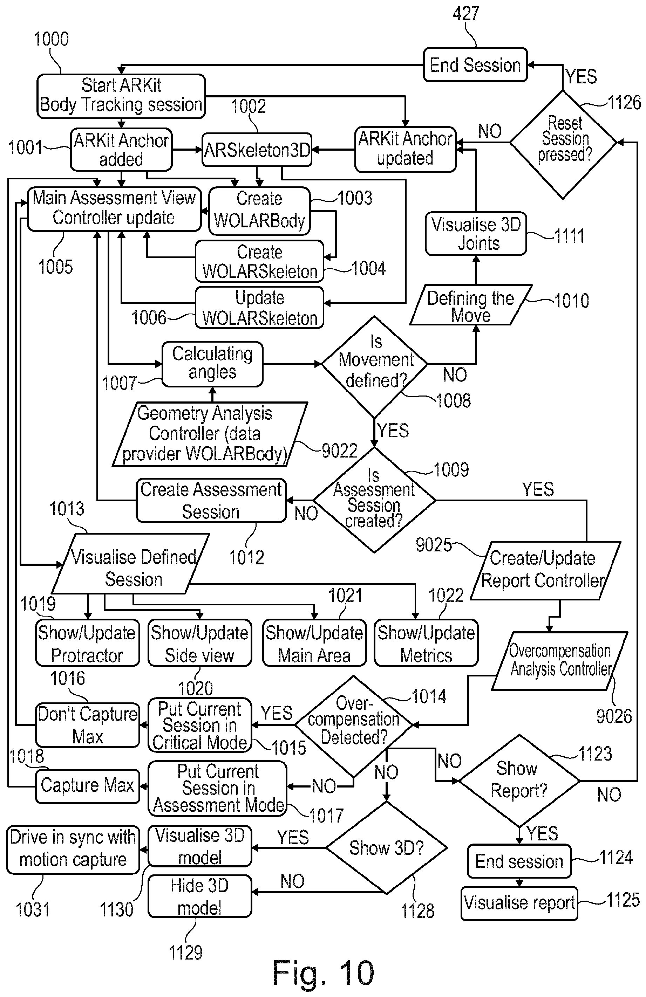

Referring to Figure 10 herein, there is illustrated schematically a process view for carrying out an assessment session. In Figure 10, all items commencing ARKit are known Apple^{®} entities, and all other entities and processes shown are devised by the inventors. All processes shown or described in Figure 10 run in parallel, unless otherwise stated.

In process 1000, ARKitBodyTracking session commences which starts all other processes running. WOLARSessionManager 9007 runs a body tracking configuration 1002 and extracts ARKit Anchors in process 1003. AR technology is based on anchors, whereby ARKit looks at a scene in first 3D coordinate space, for example a clinic or room where the subject is to perform their exercises and movements and looks for real objects which can be identified by lines or angles, for example doors, walls, floors, tables or the like, and in particular for the present embodiments and methods, looks for a human or other animal in the 3D space.

WOLARSessionManager 9007 runs continuously, waiting for the appearance of an identified anchor, i.e. a human subject within the field of view of the camera and LiDAR camera and then activates CA Analysis 9002, CA Tools 9001 and CA Output 9003.

ARKit Anchor 1003 feeds LiDAR derived data points into ARSkeleton3D 9006, producing a set of data points in first 3D coordinate space which are updated at 60 frames per second.

When an assessment session commences a defined movement is identified at process. WOLARSessionManager creates an instance of WOLARBody, which receives data from ARSkeleton3D 9006, and a set of calculations is performed to convert from first 3D coordinate space to second 3D coordinate space. WOLARSessionManager creates an instance of WOLARSkeleton 9011which provides data describing a set of dots in second 3D coordinate space. WOLARSkeleton comprises the ARSKeleton3D dataset transposed by a set of calculations from first 3D coordinate space to second 3D coordinate space.

Data outputs from WOLARBody 9010, WOLARSkeleton 9011 are input into Main Assessment View Controller 9013. The WOLARSkeleton dataset is continuously updated in real-time and updates 1006 are input into Main Assessment View Controller 9013. The updates are processed in the same way as the original creation of the WOLARSkeleton entity, and so once the WOLARSkeleton entity 1004 is created, it does not need to be further created, only updated with new data, which also needs to be processed in the same way as the original dataset for conversion from first 3D coordinate space to second 3D coordinate space. Updates occur at 60 updates per second. The result of creating 1003, 1004 the WOLARBody entity 9010, the WOLARSkeleton entity 9011, and continuously updating the WOLARSkeleton dataset is that a continuous stream of dots in second 3D coordinate space is available for further processing and analysis. The data is converted into the main class Main Assessment View Controller 9013 which is used for further analysis.

For every update of dots in second 3D coordinate space, which occurs in the best mode at 60 datasets per second, a parallel process of calculating angles 1007 occurs. Calculation of angles is performed by the Geometry Analysis Controller 9022 every time an update occurs. The calculated angles include calculation of angles between different groups of dots, each group of dots representing a different anatomical feature. For example an angle between a first group of dots representing an elbow and the second group of dots representing a spine may be calculated. Similarly, calculations of angles between each bone of an index finger are calculated in real-time, or the angle between a thigh / femur and a calf of a leg may be calculated. Additionally, calculations of the speed of change of the angles, the relative speed of motion in second 3D coordinate space of the groups of dots representing anatomical features, and the relative positions of the groups of dots representing anatomical features are also determined by Geometry Analysis Controller 9022, and automatic assessment detection module 9021 compares that data with prestored records in Medical Database 9020 to see if a known (i.e. pre-defined and stored) movement type or exercise can be identified at process 1008. This question is also implemented as "Is Assessment Session created?" 1009.

If a known pre-defined movement type or exercise cannot be identified in the medical database 9020 corresponding to the movements represented by the angles and other data supplied by the Geometry Analysis Controller 9022, then an assessment session is not created, and (optionally) instead a new movement type can be defined in Medical Database 9020 at process 1010. The three-dimensional joints can be visualized 1011 by displaying the dots on the screen overlaying the video of the subject, which is done only if a movement is not defined. If the movement is defined, a defined session can be visualised at process1013 using protractors and other views, and the visualized dots 1111 on the human body are unnecessary.

Defining a new movement is optional. If a new movement type is not to be defined at process 1010, then the Main Assessment View Controller keeps circling the loop items 1001, 1002, 1003, 1004, 1007 1008, 1010, 1111 keeping analysing the dots in WOLARSkeleton until a defined movement is recognised by Automatic Assessment Detection Module 9021 at process 1008.

When a defined movement is recognised, a new Assessment Session is created at process 1012, and the main assessment view controller 1005 can initiate visualisation of the defined session 1013, using the visualisation modules in CA Tools to create protractors, metric views and 3D animations.

Create Assessment Session 1012 occurs only once for an assessment session and subsequent querying of Is Movement Defined 1008 results in "YES" and subsequent querying of Is Assessment Session created? 1009 also results in "YES", and the Report Analysis Controller 9025 starts to record data for the session. The report analysis controller 9025 is activated only once during an assessment session, once an assessment session is created 1025, and thereafter continually updates during the same assessment session. An operating instance of the Report Controller can only exist when an Assessment Session is created 1012.

Overcompensation Control module 9026 also activates once an assessment session is created. Once activated, overcompensation control continually monitors to see if any of the movement parameters of the subject are outside predetermined limits stored in medical database 9020.

If any overcompensation is detected in process 1014, then the current assessment session is put in a Critical Mode at process 1015 and capturing of parameters by the report analysis controller 9025 is inhibited 1016 by an overcompensation alert signal generated by the overcompensation controller 9026. The critical mode causes the Metric View displays to disappear from the screen view, and a protractor for the measurand or parameter which is outside predetermined limits for that movement type is highlighted on screen, for example by turning the protractor a different colour, along with any associated dial / speedometer display relating to that parameter. Critical Mode also causes generation of an alert signal and instruction indicating to the user the error that the subject is making in performing the exercise, for example "Keep Spine Straight". The session stays in critical mode until the overcompensation controller module 9026 determines that the previously out of limit parameter is now within the predetermined limits contained within the medical database 9020 four that type of movement or exercise, at which point the overcompensation control module 9026 cancels the overcompensation alert signal and the critical mode status is lifted, the report analysis controller 9025 recommences saving data of the session, and the visualisation components recommence generating displays for the metric views, protractors and other views.

If no overcompensation is detected at process stage 1014, the current session is in assessment mode 1017, which means that data is captured 1018 and recorded by the report analysis controller module 9025, and that all appropriate metrics views, protractors, 3D Views are displayed on screen. Usually at the beginning of an assessment session, the subject does not overcompensate their movements, and there is no overcompensation detected at process 1014. However, later on during a movement or exercise, the subject may extend their movement beyond the predetermined limits for that type of exercise or movement, which triggers overcompensation detection, and activates a critical mode as herein described.

Capture Max process 1018 captures the maximum extent of movement, for example a maximum angular movement of a body part. Don't Capture Max process, 1016 which occurs during a critical mode, i.e. when overcompensation of the subject movement is detected means to any angle data or other parameters relating to a group of dots representing the subjects anatomical feature is not recorded, because it is not true maximum value for that parameter, as it was obtained when the subject was adopting a posture or position which was outside of the allowable parameters for that type of movement, for example if the subject had bent their spine outside allowable limits compared to vertical.

Whenever angles or other geometric parameters are being calculated by Geometry Analysis Controller 9022, then unless the display is inhibited by a Critical Mode condition, then parameters are visualised on the screen in real time 1013, by showing and/or updating the protractor views 1019, showing or updating the side view 1020, showing or updating the main area on the screen 1021 and showing and/or updating individual metrics 1022. Visualisations are updated 60 times per second as the incoming ARSkeleton3D dot set is refreshed at that rate.

The assessment session continues, and at any time during the session, the user has the option at 1123 of showing the report of the assessment session at process 1123. If the user chooses to show the report, that results in ending the session in process 1124, which leads to a visualisation of the final assessment session report in process 1125. Ending the session in End Session process 1124, means that data is no longer captured, and the assessment report for the current session is visualised.

Alternatively, if the user does not wish to show the report, at any time the user can reset the session at process 1126, in which case the assessment session is ended in process 1127. If the session is reset at 1126 without showing a report, then the session is ended at 1127 and data ceases to be captured by report analysis controller 9025, but there is no final assessment report for the user to view.

If no overcompensation is detected in process 1014, at any time the user can request that the 3D View is shown by selecting an on-screen command in process 1128. If the 3D View is not selected, then the 3D animation is hidden in process 1129. If the user selects that the 3D View animation model is to be displayed in process 1128, the 3D model is visualised in process 1130, and the WOLARSessionManager module 9007 drives the 3D View animation to move in synchronisation with the subject's movements in process 1131. That is, the WOLARSessionManager sends the relevant angle data, and other geometric data to the 3D animation engine, updated 60 times per second, and the animation engine returns a rendered image of the selected anatomical feature corresponding to the movement type being carried out which is displayed by 3D View component 9031, where the movement of the animated 3D model is synchronized to the movement of the subject in first 3D coordinate space. The 3D view enables the user to see which muscles are involved in the exercise, and the 3D view can be rotated to see a side view of the dissected 3D animated model.

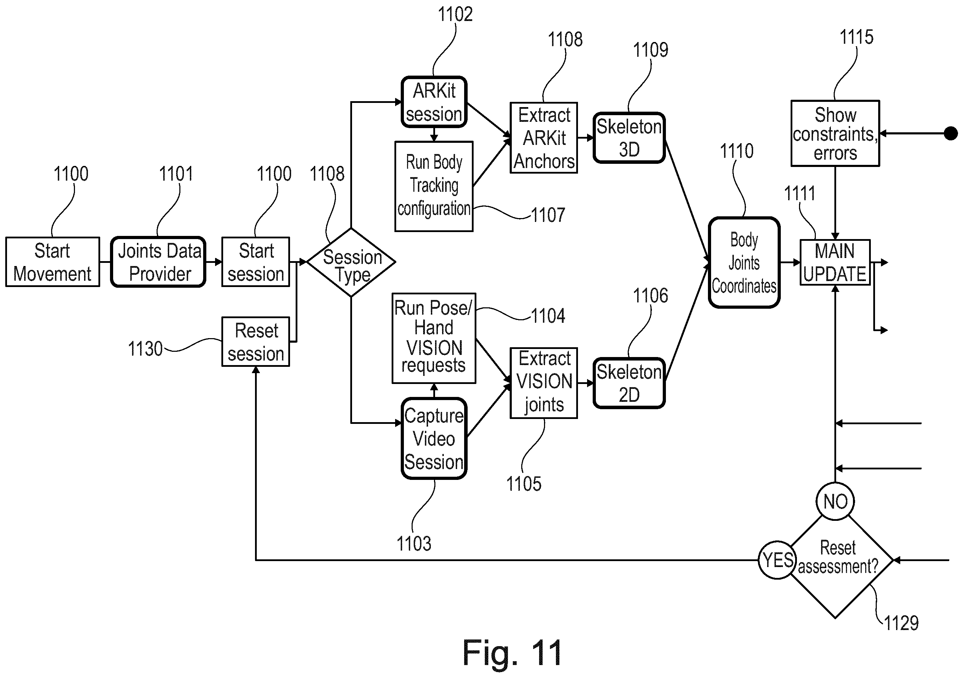

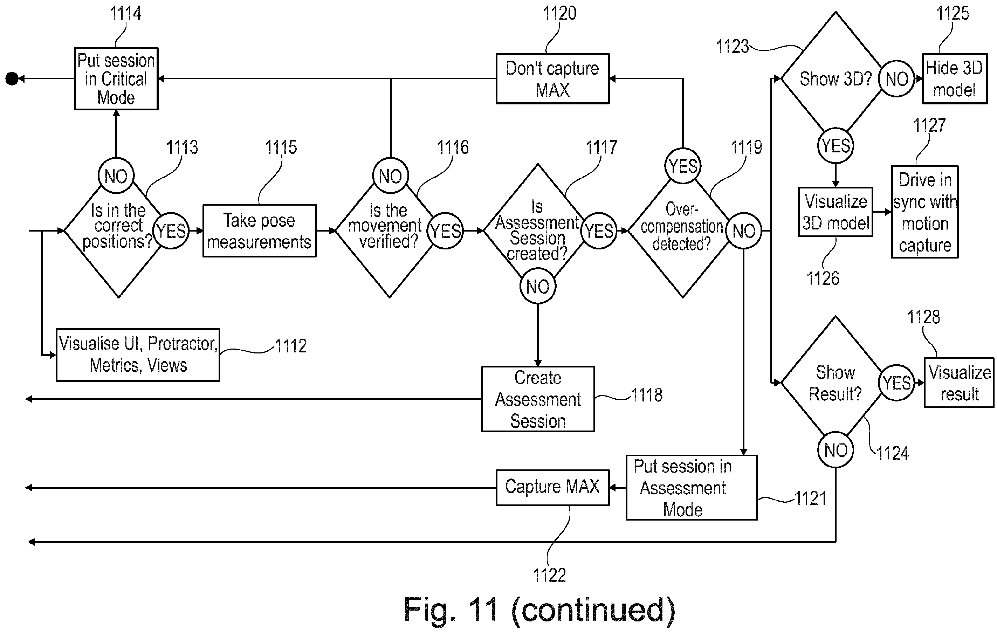

Referring to Figure 11 herein, there is illustrated schematically a further process flow according to a specific method herein. The logical entities shown in Figure 11 are each ongoing processes ongoing processes which run continuously in real-time unless otherwise stated, or less inhibited by other processes within the process suite shown.

A person in the field of view of the LiDAR camera starts a movement 1100 which starts a session. A session type 1101 can be an ARKit session 1101, and/ or a Capture Video session 1103. Starting a capture video session 1103 runs Pose / Hand VISION requests 1104, and extracts VISION joints 1105 resulting in two-dimensional skeleton data 1106.

Starting an ARKit session 1102 runs a body tracking configuration process 1107 which extracts ARKit anchors, and results in three-dimensional skeleton data Skeleton 3D 1109.

The two-dimensional skeleton data Skeleton 2D and the three-dimensional skeleton data Skeleton 3D combines into Body Joint Coordinates data 1110.

A main update process 1111 results in generation of visualisation, protractors, metrics and views 1112. A main update 1111 leads to the process determining 1113 whether the subject is in a correct position or not. If the subject is not in a correct position, the process puts the session in a critical mode 1114 and shows 1115 the constraints and errors of the subject, resulting in inhibition of views and displays as described herein.

If the subject is determined to be in a correct position or pose 113, the process goes on to take pose measurements 1115 and determines 1116 whether those pose measurements can be verified as a particular type of movement at process 1116. If the pose measurements do not coincide with the verified movement, then the process puts the session in a critical mode 1114 and shows constraints and errors 1115 which are displayed at a main update 1111.

If the movement is verified, the process determines 1117 whether an assessment session has been created, and if no assessment session has already been created, at process 1118 the system creates an assessment session which prompts a main update process 1111.

If an assessment session has already been created, an assessment session already exists and the process monitors whether there is any Overcompensation Detected at process 1119. If at any time during the session overcompensation is detected, capturing of maximum movement parameters is inhibited at process 1120. However as long as overcompensation is not detected, the apparatus continues to record three-dimensional skeleton data and video and the session is put in an Assessment Mode 1121 in which maximum movement parameters are captured in process 1122. Captured maximum movement parameters of the subject are fed into main update 1111.

Provided there is no overcompensation detected at process 1119, three-dimensional coordinates can be shown at process 1123 and results can be shown at process 1124.

If the user does not wish to show three-dimensional data, the three-dimensional model can be hidden at process 1124. If the user selects to show three-dimensional data, then a three-dimensional model is visualised at process 1126 and the three-dimensional visualization is driven in synchronisation with motion capture in process 1127.

If the user selects to visualise results at process 1124 the results are visualised in process 1128.

Selecting to show results in process 1124 gives the option to reset an assessment session in process 1129. If a user selects to reset an assessment session, then a reset is made at process which then goes on to determine a session typing process 1101 as hereinbefore described. If a user does not activate a reset assessment, then main updates continue to be created in process 1111.

The following logical entities, processes, commands and queries are present in the logical processes of Figure 11 herein:
Show 3D?
Hide 3D model
Show constraints, errors
Put session in critical
Mode
Don't capture
MAX
NO
ARKit session
Extract ARKit Anchors
Skeleton 3D
YES
Run Body Tracking configuration
NO
NO
YES
Visualize 3D
Model
Drive in sync with motion capture
Is in the correct position?
Is the movement verified?
Is AssessmentSession created?
Overcompensation detected?
Session Type
MAIN UPDATE
Take pose measurements
Start Movement
Joints Data Provider
Start session
Body Joints Coordinates
No
No
YES
Show Result?
Run Pose/Hand VISION requests
NO
Visualise result
Reset Session
Extract VISION joints
Skeleton 2D
YES
Visualize UI , Protractor, Metrics, Views
Capture Video Session
NO
Create Assessment Session
Capture MAX
Put session in Assessment Mode
NO
Reset assessment?
YES

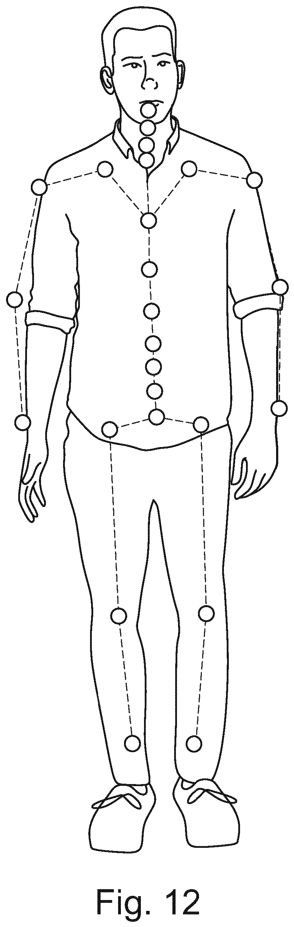

Referring to Figure 12 herein, there is shown schematically a view showing a two-dimensional screen image of a subject present in first coordinate space, with a two-dimensional view of a plurality of 3 dimensional points in first coordinate space, the three-dimensional points being shown as dots superimposed onto the two-dimensional image of the subject.

In Figure 12, the plurality of three dimensional points are provided as the ARSkeleton3D data which track approximate skeletal / anatomical features of the subject in first coordinate space. As the subject moves in the first coordinate space the stream of dots approximately track the movements of the subject's main anatomical features, including limbs, spine. A set of 93 individual dots are received, the 3D coordinates of each dot being updated 60 times per second. The dots comprise the data class ARSkeleton3D.

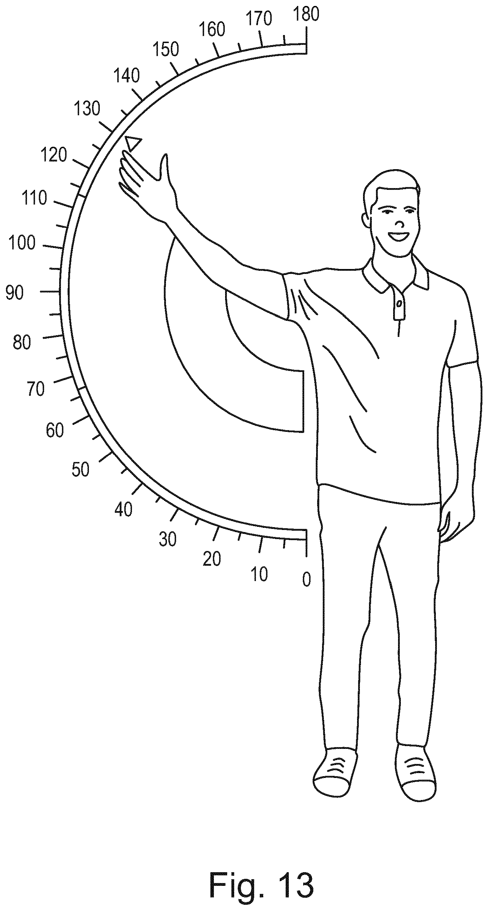

Referring to Figure 13 herein, there is illustrated schematically one example of a protractor display 1300 generated from Protractor visualisation module 9036 using data output from Virtual Protractors module 9023. The protractor display comprises a semicircle having angular graduations. In three-dimensional coordinate space, the protractor comprises a semi-circle having a main central axis coincident with a horizontal plane in the three-dimensional coordinate space. A diameter on one side of the semicircle remains horizontal in three-dimensional coordinate space. The orientation of the scale of the virtual protractor follows a group of dots representing the arm of the subject, so that as the subject swings their arm in the first coordinate space, a main plane coinciding with the semicircular protractor scale moves around in the second three-dimensional coordinate space so that the main plane of the protractor display remains substantially coincident with a line of dots representing the subject's arm.

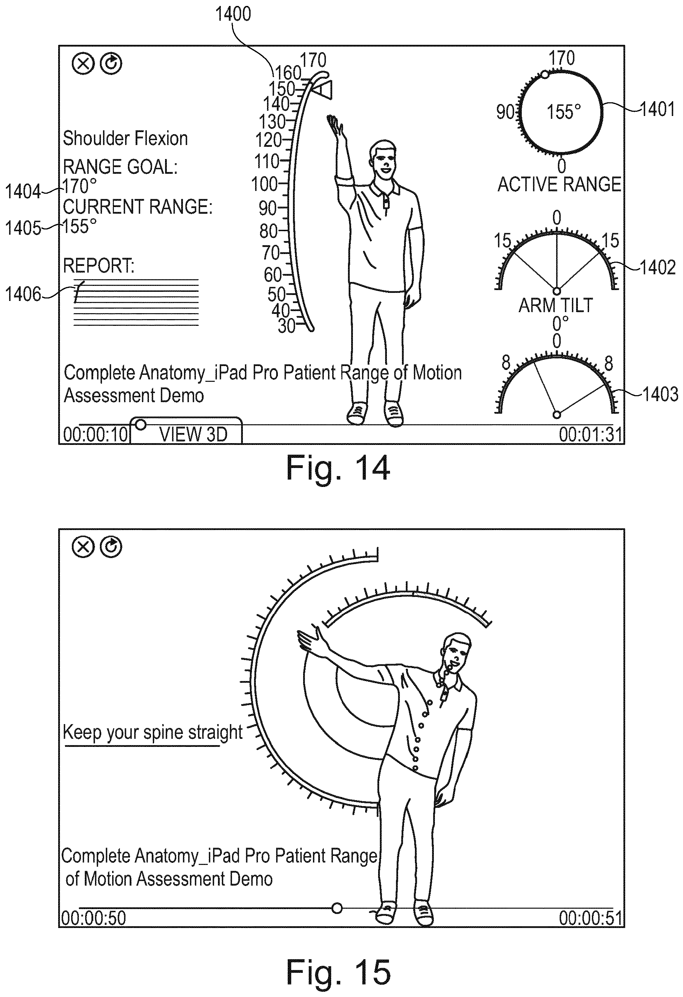

Referring to Figure 14 herein, there is shown schematically a screen view of a subject on the user interface of the device. An image of the subject is displayed as a two-dimensional video image. Superimposed on the two-dimensional image there is a protractor image 1408 plurality of metric view images 1402-1406. The protractor image is generated from data output from the Virtual Protractors module 9023 and protractor visualisation module 9036. The metric views are generated from data output from the metric views (speedometer's) module 9024 and visualised by the metric views visualisation module 9035. In the example shown a first metric view 1401 shows a circle having an angular graduation with a movable marker icon which traverses around a circular path according to an angle of movement of an anatomical feature, in this case shoulder angle in a shoulder flexion movement. The circular display is split into a left half as viewed by the user, corresponding to a right shoulder of the subject facing the camera and LiDAR device, and a right half as viewed by the user on the screen, corresponding to a left shoulder of the subject when the subject is facing the camera and LiDAR camera of the device. When the subject raises their right arm, the corresponding (left) side of the circular Active Range display shows in real time and angle of movement. The circular dot icon represents the instantaneous angle adopted by the right shoulder, and a circular ribbon indicates the maximum range of angular movement achieved by the shoulder during the current exercise undertaken by the subject. There is also a numerical display displaying the current instantaneous angle of flexion of the shoulder.

A second metric display 1402 indicates on a circular dial type review and angle of arm tilt. The second metric display view comprises a rotatable needle pivoted around the central point of the circular display, with the needle moving to anticlockwise for arm tilt in one direction and clockwise to represent arm tilt in another opposite direction.

A third metric display 1403 comprises a circular graduated dial display having a rotatable needle, rotatable about a central point of the circular display for showing spinal alignment. The needle pointing vertically upwards indicates that the spine is aligned vertically. If the needle is tilted to the left side as viewed by the user, this corresponds with the alignment of the subject's spine tilting to one side, and if the needle is tilted to the opposite, right side that corresponds with the alignment of the subject spine tilting to the opposite side. The degree of tilt angle from the vertical is indicated in degrees on the graduated scale around the perimeter of the circular dial display.

A fourth metric view display 1404 displays a numerical goal for range of movement of shoulder flexion, in this case 170°. A fifth metric view display 1405 displays a currently achieved angular range of movement of shoulder flexion, in this case 155°. A sixth metric display 1406 gives a graph report of shoulder flexion angle on a vertical Y axis against time on a horizontal X axis. The graph completes in real time as the subject moves their arm.

All of the above metric views are calculated dynamically in the Metric Views generation module 9024 and displays are generated dynamically by the Metrics View visualisation module 9035.

Referring to Figure 15 herein, there is illustrated schematically one example of a protractor display which is inhibited when Overcompensation Controller 9026 generates an overcompensation alert signal. The display screen is inhibited so that all metric views previously shown in Figure 13 are disabled and not shown, with the exception of the spinal alignment display 1303 which shows that the spine of the subject is out of alignment for the shoulder flexion movement being carried out. The protractor view turns a different colour (preferably red) and a message "keep your spine straight" is generated and displayed on the screen. Additionally, a line of dots representing the subject's spine is superimposed on the video display of the subject, showing an approximate position of the subject's spine.

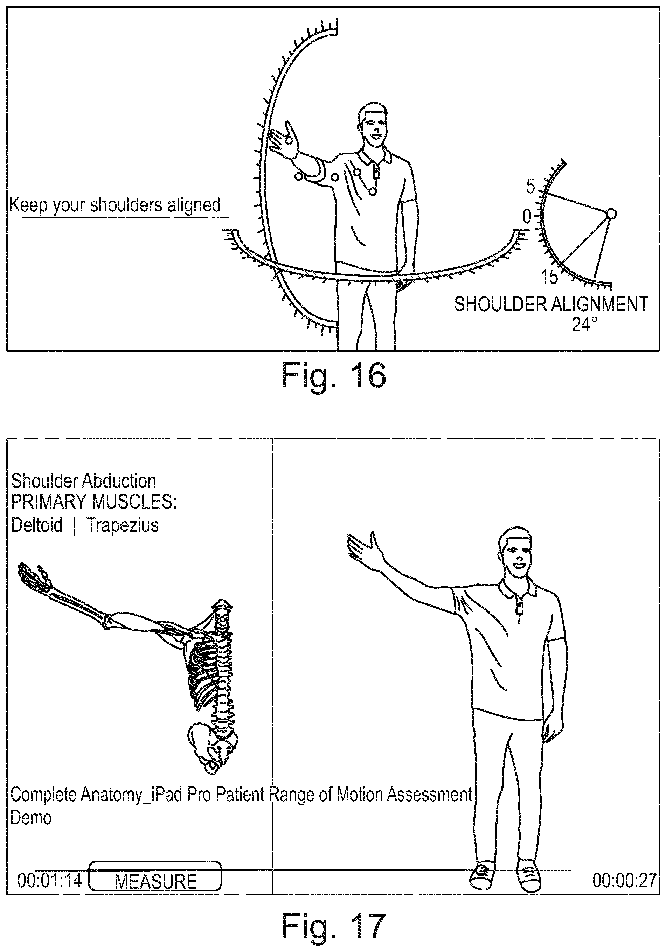

Referring to Figure 16 herein, there is illustrated schematically a second example of a screen view having a protractor display when overcompensation controller 9026 generates an overcompensation alert signal. Compared to the metric views displayed in Figure 14 herein, the subject's shoulders have become out of alignment resulting in detection of the misalignment by overcompensation controller 9026, resulting in all of the metric views except the shoulder alignment dial display being inhibited from being shown. The relevant metric display for the parameter which is out of alignment, in this case, the shoulder alignment display is generated and shown, along with an upright protractor 1600 and a lateral protractor 1601 being displayed, the lateral protractor in this case showing that the subject has move their arm too far forward towards the front of their body, which is outside the allowable parameters for a correct exercise for shoulder flexion. The value of shoulder alignment is shown on the metric view for shoulder alignment, in this case being outside of goal parameters, and the lateral protractor is highlighted in a colour which indicates an out of goal parameter condition, in this example being highlighted in red.

Referring to Figure 17 herein, there is illustrated schematically a screen view comprising a main view showing a video of a subject, generated by Main View visualisation module 9034 and a digitally generated 3D anatomical view generated by 3D View module 9031. The main view occupies a first area of the screen, and the 3D View occupies a second area of the screen. The 3D View is generated externally from a prior art system. WOLARSessionManager module 9007 sends data describing the required 3D view to the known 3D View generator, including data describing: the type of movement which is being undertaken; the individual anatomical features which are desired to be shown, for example the particular muscles and bones required in the 3D View; data describing the relative angles and relative orientations of each of those anatomical features in second 3D coordinate space. The known 3D View generation module returns rendered images to the WOLARSession manager, which are then displayed via the 3D View visualisation module to generate the visible screen display. As the user moves their arm in real time, the 3D View is continually updated and refreshed at 60 frames per second, so that the anatomical representation provided by 3D view follows movement of the subject in real time, adopting the same, or substantially the same positions and angles of anatomical features as the subject.

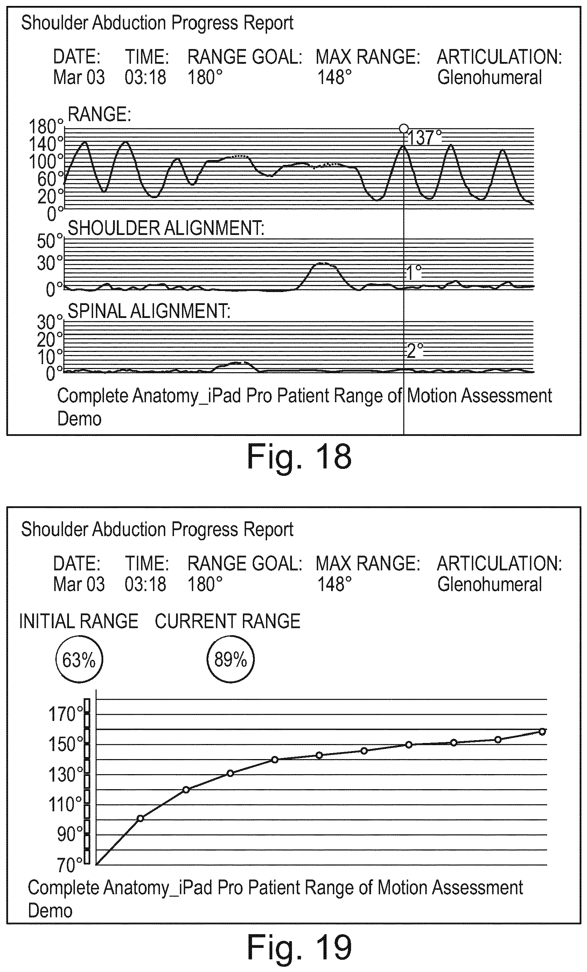

Referring to Figure 18 herein, there is illustrated schematically one example of a progress report generated by Report Analysis Controller module 9025, in this example a shoulder abduction progress report. The progress report comprises three graphical displays corresponding to:
- Range - a graphical display of angular range of movement of an anatomical feature on an upright axis (y axis), against time on a horizontal axis;
- Shoulder Alignment - a graphical display of angle of shoulder alignment on an upright axis, against time on a horizontal axis;
- Spinal Alignment - a graphical display of angle of spinal alignment on a vertical axis, against time on a horizontal axis.

For each graphical display, a horizontally moving upright cursor tracks the instantaneous value of the parameter being measured and displays a numerical value of that parameter. Also displayed are other metrics generated by Metrics View component 9035 including date, time, range goal, maximum range, articulation (the muscle or muscle groups which are being articulated).

Referring to Figure 19 herein, there is illustrated schematically a graphical report containing data generated by the Report Analysis Controller 9025, the display of the graphical report being generated by the Assessment Report visualisation module 9032. The graphical report in this example comprises a two-dimensional chart having a horizontal time axis (x axis) and a vertical and angle of movement axis (y axis). As the subject performs an exercise in real-time, individual measurements of angle of a body part, in this case a subject's arm, are plotted at periodic time intervals during an exercise session. The Report Analysis Controller supplies all the basic data parameters, and the Assessment Report visualisation module 9032 inserts the data into a predetermined report format an example of which is as shown in Figure 19. There may be applied a subroutine which joins up individual data points using lines, in order to create a line graph. Alternatively, the Assessment Report visualisation module may have other preset report formats, for example bar chart formats.

Additional data which may be visualised on the graphical report comprises:
- Name of movement or exercise, for example "shoulder abduction";
- Date
- Time
- range goal in degrees, being the ideal range of movement for a particular movement or exercise;
- Maximum range - the maximum range of movement actually achieved by a subject during the session;
- Articulation - the name of the muscle or muscle groups which are being articulated during the movement, for example the Glenohumeral muscle;
- Initial range - an initial range of movement achieved during an exercise session;
- Current range - a current range of movement being achieved during an exercise session.

### Modifications and Variations

In the present best mode implementation, sessions are not stored to a database, and the data is deleted when the session ends. However in in alternative embodiments the WOLARSession Manager may interface with a communications port of the device to wirelessly transmit all of the session data to a remote data storage device or remote database for remote storage and subsequent processing.

The present best mode, the apparatus is optimised for use on a hand held computing platform of the tablet type. However in the general case the specific method herein may be carried out on any hardware platform having the basic components shown in Figure 8 herein, and provided that the speed of data connection within the computing platform is sufficiently fast and has high enough bandwidth, various ones of the modules as shown in Figure 9 herein may be implemented on a distributed computing platform.

In the above specific embodiments and methods, there is shown an example of a human subject, but the specific embodiments and methods may find use in veterinary surgery with appropriate variation of the individual movement and exercise types stored in medical database 9020.

### Advantages

In the above specific embodiments, there are the following advantages compared to various prior art goniometers:
- with the presently presented embodiments and methods, there is effectively an easy-to-use plug and play system for taking range of movement measurements;
- all measurements required for determining range of motion of a limb or joint are taken by a single device;
- the device disclosed herein does not have to be strapped to, held against or otherwise attached to the person whose range of movement is being determined;
- the device disclosed herein can be used by a single user, who is also the person whose range of movement is being determined;
- more conveniently, the device disclosed herein may be operated by a second person, who need not be a qualified medical personnel;
- there is minimal set up procedure required to use the presently disclosed device, compared to prior art electronic goniometer devices which require a more lengthy setup and initial referencing procedure;
- due to the minimal setup procedure there is a short learning curve for a user determining how to use the specific embodiments disclosed herein and a user can be up and running to use the embodiments very quickly with little or no training.
- The overcompensation control functionality described herein allows the embodiments to determine whether an exercise or motion is being carried out correctly. Previously, in the prior art only a doctor or physiotherapist could determine if a subject was carrying out a movement or an exercise correctly. The exercise correctly. Prior art electronic goniometers do not provide the functionality of determining if a subject is carrying out a movement or exercise correctly, and do not collect enough information to make such an assessment;
- for each type of exercise or movement, multiple parameters can be displayed simultaneously in real time, using a variety of visual displays, for example graphical displays, odometer type displays, and protractor displays, enabling ease-of-use, enhanced information availability, and enhanced information visualisation compared to prior art goniometer devices;
- motion capture in general as a technical concept has been known for many years, but generally requires a studio with tracking monitors and body suits having sensors. It has not been previously applied in a portable handheld device for goniometer measurements measuring human or animal movement parameters at a point-of-care on a single device without any setup and without the need for any specialist knowledge on the part of the user.

## Claims

1. A method of making goniometer angle measurements between anatomical parts of a human subject by processing data describing anatomical features of said subject;
said data comprising a plurality point coordinates (1002) in a first three dimensional coordinate space, said plurality of point coordinates representing anatomical features of said subject;
said method comprising:
determining (9022) one or more geometric parameters of said plurality of point coordinates;
grouping (9022) said plurality of point coordinates into groups of points, each of which represents a said anatomical feature;
recognising (9022) a said anatomical feature from said determined geometric parameters;
comparing said determined geometric parameters with a set of pre-determined geometric parameters stored in a database (9020), each said set of pre-determined geometric parameters corresponding to a respective pre-stored movement type for said recognised anatomical feature stored in said database;
depending on a result of said comparison, identifying (1008, 9021, 9022) a movement of said plurality of point coordinates with a said pre-stored movement type;
generating (1007) a measurement of an angle of said recognised anatomical feature; and **characterized in that** the method further comprises:
generating, on a user interface, a real time protractor display (9036) for displaying an angle between at least two said groups of point coordinates.

2. The method as claimed in claim 1, further comprising:
comparing (9026) a posture of said subject, by comparing an orientation of a first set of three-dimensional coordinates representing an anatomical feature with a stored reference orientation (9022) for said anatomical feature; and
identifying (1014) whether said set of three-dimensional coordinates have an orientation which is outside a predetermined range of orientations relative to said reference orientation.

3. The method as claimed in any one of the preceding claims, further comprising capturing (9000) a video image of said subject in said three-dimensional coordinate space;
generating a protractor (9036) which moves in real time corresponding with a movement of said subject; and
overlaying (1013, 1019) said protractor on said video image (9030).

## Patentansprüche

1. Verfahren zum Durchführen von Goniometer-Winkelmessungen zwischen anatomischen Teilen eines menschlichen Subjekts durch Verarbeiten von Daten, die anatomische Merkmale des Subjekts beschreiben;
wobei die Daten eine Vielzahl von Punktkoordinaten (1002) in einem ersten dreidimensionalen Koordinatenraum umfassen, wobei die Vielzahl von Punktkoordinaten anatomische Merkmale des Subjekts darstellt;
wobei das Verfahren Folgendes umfasst:
Bestimmen (9022) von einem oder mehreren geometrischen Parametern der Vielzahl von Punktkoordinaten;
Gruppieren (9022) der Vielzahl von Punktkoordinaten in Gruppen von Punkten, von denen jeder ein anatomisches Merkmal darstellt;
Erkennen (9022) eines anatomischen Merkmals aus den bestimmten geometrischen Parametern;
Vergleichen der bestimmten geometrischen Parameter mit einem Satz von vorbestimmten geometrischen Parametern, der in einer Datenbank (9020) gespeichert ist, wobei jeder Satz von vorbestimmten geometrischen Parametern einem jeweiligen vorgespeicherten Bewegungstyp für das erkannte anatomische Merkmal entspricht, das in der Datenbank gespeichert ist;
abhängig von einem Ergebnis des Vergleichs, Identifizieren (1008, 9021, 9022) einer Bewegung der Vielzahl von Punktkoordinaten mit dem vorgespeicherten Bewegungstyp;
Generieren (1007) einer Messung eines Winkels des erkannten anatomischen Merkmals; und
**dadurch gekennzeichnet, dass** das Verfahren ferner Folgendes umfasst:
Generieren, auf einer Benutzerschnittstelle, eines Echtzeit-Protraktor-Displays (9036) zum Anzeigen eines Winkels zwischen zumindest zwei Gruppen von Punktkoordinaten.

2. Verfahren nach Anspruch 1, ferner umfassend:
Vergleichen (9026) einer Haltung des Subjekts durch Vergleichen einer Ausrichtung eines ersten Satzes von dreidimensionalen Koordinaten, die ein anatomisches Merkmal darstellen, mit einer gespeicherten Referenzausrichtung (9022) für das anatomische Merkmal; und
Identifizieren (1014), ob der Satz von dreidimensionalen Koordinaten eine Ausrichtung aufweist, die außerhalb eines vorbestimmten Bereichs von Ausrichtungen relativ zu der Referenzausrichtung ist.

3. Verfahren nach einem der vorhergehenden Ansprüche, ferner umfassend Aufnehmen (9000) eines Videobildes des Subjekts in dem dreidimensionalen Koordinatenraum;
Generieren eines Protraktors (9036), der sich in Echtzeit entsprechend einer Bewegung des Subjekts bewegt; und
Überlagern (1013, 1019) des Protraktors auf dem Videobild (9030).

## Revendications

1. Procédé permettant d'effectuer des mesures d'angle goniométrique entre des parties anatomiques d'un sujet humain en traitant des données décrivant des caractéristiques anatomiques dudit sujet ;
lesdites données comprenant une pluralité de coordonnées de point (1002) dans un premier espace de coordonnées tridimensionnelles, ladite pluralité de coordonnées de point représentant des caractéristiques anatomiques dudit sujet ;
ledit procédé comprenant :
la détermination (9022) d'un ou de plusieurs paramètres géométriques de ladite pluralité de coordonnées de point ;
le regroupement (9022) de ladite pluralité de coordonnées de point en groupes de points, dont chacun représente une dite caractéristique anatomique ;
la reconnaissance (9022) d'une dite caractéristique anatomique à partir desdits paramètres géométriques déterminés ;
la comparaison desdits paramètres géométriques déterminés à un ensemble de paramètres géométriques prédéterminés mémorisés dans une base de données (9020), chaque dit ensemble de paramètres géométriques prédéterminés correspondant à un type de mouvement pré-mémorisé respectif pour ladite caractéristique anatomique reconnue mémorisée dans ladite base de données ;
en fonction d'un résultat de ladite comparaison, l'identification (1008, 9021, 9022) d'un mouvement de ladite pluralité de coordonnées de point avec un dit type de mouvement pré-mémorisé ;
la génération (1007) d'une mesure d'un angle de ladite caractéristique anatomique reconnue ; et **caractérisé en ce que** le procédé comprend en outre :
la génération , sur une interface utilisateur, d'un affichage de rapporteur en temps réel (9036) pour afficher un angle entre au moins deux desdits groupes de coordonnées de point.

2. Procédé selon la revendication 1, comprenant en outre :
la comparaison (9026) d'une posture dudit sujet, en comparant une orientation d'un premier ensemble de coordonnées tridimensionnelles représentant une caractéristique anatomique avec une orientation de référence mémorisée (9022) pour ladite caractéristique anatomique ; et
l'identification (1014) du fait que ledit ensemble de coordonnées tridimensionnelles comporte, ou non, une orientation qui est en dehors d'une plage prédéterminée d'orientations par rapport à ladite orientation de référence.

3. Procédé selon l'une quelconque des revendications précédentes, comprenant en outre la capture (9000) d'une image vidéo dudit sujet dans ledit espace de coordonnées tridimensionnelles ;
la génération d'un rapporteur (9036) qui se déplace en temps réel en correspondance avec un mouvement dudit sujet ; et
la superposition (1013, 1019) dudit rapporteur sur lad(te image vidéo (9030).
